# EUROPÄISCHE PATENTSCHRIFT

(11) **EP 1 443 822 B1**
(45) Veröffentlichungstag und Bekanntmachung des Hinweises auf die Patenterteilung: **17.12.2008**
(21) Anmeldenummer: 02774735.1
(22) Anmeldetag: 21.10.2002
(51) Int. Cl.: A01N 47/38, C07D 409/12, C07D 513/04, C07D 498/04, C07D 487/04

(54) **SUBSTITUIERTE THIEN-3-YL-SULFONYLAMINO(THIO)CARBONYL-TRIAZOLIN(THI)ONE**
SUBSTITUTED THIEN-3-YL-SULFONYLAMINO(THIO)CARBONYL-TRIAZOLIN(THI)ONES
THIEN-3-YL-SULFONYLAMINO(THIO)CARBONYL-TRIAZOLIN(THI)ONES SUBSTITUEES

(30) Priorität: 02.11.2001 DE 10154074
(43) Veröffentlichungstag der Anmeldung: 11.08.2004
(73) Patentinhaber: Bayer CropScience AG, 40789 Monheim (DE)
(72) Erfinder: GESING, Ernst-Rudolf F., 40699 Erkrath (DE); DREWES, Mark, Wilhelm, 40764 Langenfeld (DE); DAHMEN, Peter, 41470 Neuss (DE); FEUCHT, Dieter, 65779 Kelkheim (DE); PONTZEN, Rolf, 42799 Leichlingen (DE)
(74) Vertreter: Krieg, Robert Alexander
(86) Internationale Anmeldenummer: PCT/EP2002/011743
(87) Internationale Veröffentlichungsnummer: WO 2003/037086

(56) Entgegenhaltungen:
- WO-A-01/05788
- WO-A-97/16449

## Beschreibung

Die Erfindung betrifft neue substituierte Thien-3-yl-sulfonylamino(thio)carbonyl-triazolin(thi)one, Verfahren zu ihrer Herstellung sowie ihre Verwendung als Herbizide.

Es ist bereits bekannt, dass bestimmte substituierte Thienylsulfonylamino(thio)carbonyl-triazolin(thi)one, wie z.B. die Verbindungen 4-[[[(4,5-Dihydro-3-ethoxy-4-methyl-5-oxo-1H-1,2,4-triazol-1-yl)-carbonyl]-amino]-sulfonyl]-5-methyl-3-thiophencarbonsäure-methylester, 4-[[[(4,5-Dihydro-3-methoxy-4-methyl-5-oxo-1H-1,2,4-triazol-1-yl)-carbonyl]-amino]-sulfonyl]-5-methyl-3-thiophencarbonsäure-methylester, 4-[[[(4,5-Dihydro-4-methyl-5-oxo-3-n-propoxy-1H-1,2,4-triazol-1-yl)-carbonyl]-amino]-sulfonyl]-5-methyl-3-thiophencarbonsäure-methylester, 4-[[[(4,5-Dihydro-4-methyl-5-oxo-3-i-propoxy-1H-1,2,4-triazol-1-yl)-carbonyl]-amino]-sulf onyl]-5-methyl-3-thiophencarbonsäure-methylester, 4-[[[(4-Cyclopropyl-4,5-di-hydro-3-methoxy-5-oxo-1H-1,2,4-triazol-1-yl)-carbonyl]-amino]-sulfonyl]-5-methyl-3-thiophencarbonsäure-methylester, 4-[[[(4-Cyclopropyl-4,5-dihydro-3-ethoxy-5-oxo-1H-1,2,4-triazol-1-yl)-carbonyl]-amino]-sulfonyl]-5-methyl-3-thiophencarbonsäure-methylester, 4-[[[(4-Cyclopropyl-4,5-dihydro-5-oxo-3-n-propoxy-1H-1,2,4-triazol-1-yl)-carbonyl]-amino]-sulfonyl]-5-methyl-3-thiophencarbonsäure-methylester, 4-[[[(4-Cyclopropyl-4,5-dihydro-5-oxo-3-i-propoxy-1H-1,2,4-triazol-1-yl)-carbonyl]-amino]-sulfonyl]-5-methyl-3-thiophencarbonsäure-methylester, 4-[[[(3,4-Dicyclopropyl-4,5-dihydro-5-oxo-1H-1,2,4-triazol-1-yl)-carbonyl]-amino]-sulfonyl]-5-methyl-3-thiophencarbonsäure-methylester, 4-[[[(4,5-Dihydro-3,4-dimethyl-5-oxo-1H-1,2,4-triazol-1-yl)-carbonyl]-amino]-sulfonyl]-5-methyl-3-thiophencarbonsäure-methylester, 4-[[[(4,5-Dihydro-3-ethyl-4-methyl-5-oxo-1H-1,2,4-triazol-1-yl)-carbonyl]-amino]-sulfonyl]-5-methyl-3-thiophencarbonsäure-methylester, 4-[[[(4,5-Dihydro-4-methyl-3-methylthio-5-oxo-1H-1,2,4-triazol-1-yl)-carbönyl]-amino]-sulf onyl]-5-methyl-3-thiophencarbonsäure-methylester, 4-[[[(4,5-Dihydro-3,4-dimethoxy-5-oxo-1H-1,2,4-triazol-1-yl)-carbonyl]-amino]-sulfonyl]-5-chlor-3-thiophencarbonsäure-ethylester, 4-[[[(4,5-Dihydro-3-ethoxy-4-methyl-5-oxo-1H-1,2,4-triazol-1-yl)-thioxocarbonyl]-amino]-sulfonyl]-5-fluor-3-thiophencarbonsäure-methylester, 4-[[[(4,5-Dihydro-3-ethyl-4-methoxy-5-thioxo-1H-1,2,4-triazol-1-yl)-carbonyl]amino]-sulfonyl]-5-trifluorniethyl-3-thiophencarbonsäure-methylester, 4-[[[(4,5-Dihydro-4-ethyl-3-methoxy-5-oxo-1H-1,2,4-triazol-1-yl)-carbonyl]-amino]-sulfonyl]-5-methyl-3-thiophencarbonsäure-ethylester und 4-[[[(3,4-Dimethyl-5-oxo-4,5-dihydro-1H-1,2,4-triazol-1-yl)-carbonyl]-amino]-sulfonyl]-5-ethyl-3-thiophen-carbonsäure-1-propylester, herbizide Eigenschaften aufweisen (vgl. WO-A-01/05788, vgl. auch WO-A-97/16449, WO-A-98/24787). Die Wirkung dieser Verbindungen ist jedoch nicht in allen Belangen zufriedenstellend.

Es wurden nun die neuen substituierten Thien-3-yl-sulfonylamino(thio)carbonyltriazolin(thi)one der allgemeinen Formel (I) 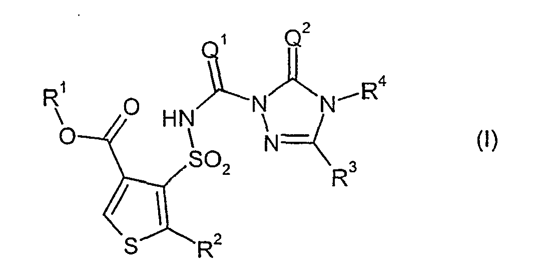 in welcher
Q¹ für O (Sauerstoff) oder S (Schwefel) steht,
Q² für O (Sauerstoff) oder S (Schwefel) steht,
R¹ für gegebenenfalls durch Cyano, Halogen oder C₁-C₄-Alkoxy substituiertes Alkyl mit 1 bis 6 Kohlenstoffatomen, für jeweils gegebenenfalls durch Cyano oder Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil, für jeweils gegebenenfalls durch Nitro, Cyano, Halogen, C₁-C₄-Alkyl oder C₁-C₄-Alkoxy substituiertes Aryl oder Arylalkyl mit jeweils 6 oder 10 Kohlenstoffatomen in der Arylgruppe und gegebenenfalls 1 bis 4 Kohlenstoffatomen im Alkylteil, oder für jeweils gegebenenfalls durch Nitro, Cyano, Halogen, C₁-C₄-Alkyl oder C₁-C₄-Alkoxy substituiertes Heterocyclyl oder Heterocyclylalkyl mit jeweils bis zu 6 Kohlenstoffatomen und zusätzlich 1 bis 4 Stickstoffatomen und/oder 1 bis 2 Sauerstoff- oder Schwefelatomen in der Heterocyclylgruppe und gegebenenfalls 1 bis 4 Kohlenstoffatomen im Alkylteil steht,
R² für Wasserstoff, Cyano, Nitro, Halogen, für jeweils gegebenenfalls durch Cyano, Halogen oder C₁-C₄-Alkoxy substituiertes Alkyl, Alkoxy, Alkoxycarbonyl, Alkylthio, Alkylsulfinyl oder Alkylsulfonyl mit jeweils 1 bis 6 Kohlenstoffatomen in der Alkylgruppe, oder für jeweils gegebenenfalls durch Cyano oder Halogen substituiertes Alkenyl, Alkinyl, Alkenyloxy oder Alkinyloxy mit jeweils 2 bis 6 Kohlenstoffatomen in der Alkenyl- oder Alkinylgruppe steht,
R³ für durch Halogen, substituiertes Alkoxy, mit 1 bis 6 Kohlenstoffatomen steht, und,
R⁴ für Wasserstoff, Hydroxy, Amino, Cyano, für C₂-C₁₀-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes Alkyl mit 1 bis 6 Kohlenstoffatomen, für jeweils gegebenenfalls durch Fluor, Chlor und/oder Brom substituiertes Alkenyl oder Alkinyl mit jeweils 2 bis 6 Kohlenstoffatomen, für jeweils gegebenenfalls durch Fluor, Chlor, Brom, Cyano, C₁-C₄-Alkoxy oder C₁-C₄-Alkoxy-carbonyl substituiertes Alkoxy, Alkylamino oder Alkyl-carbonylamino mit jeweils 1 bis 6 Kohlenstoffatomen in der Alkylgruppe, für Alkenyloxy mit 3 bis 6 Kohlenstoffatomen, für Dialkylamino mit jeweils 1 bis 4 Kohlenstoffatomen in den Alkylgruppen, für jeweils gegebenenfalls durch Fluor, Chlor, Brom, Cyano und/oder C₁-C₄-Alkyl substituiertes Cycloalkyl, Cycloalkylamino oder Cycloalkylalkyl mit jeweils 3 bis 6 Kohlenstoffatomen in der Alkylgruppe und gegebenenfalls 1 bis 4 Kohlenstoffatomen im Alkylteil, oder für jeweils gegebenenfalls durch Fluor, Chlor, Brom, Cyano, Nitro, C₁-C₄-Alkyl, Trifluormethyl und/oder C₁-C₄-Alkoxy substituiertes Aryl oder Arylalkyl mit jeweils 6 oder 10 Kohlenstoffatomen in der Arylgruppe und gegebenenfalls 1 bis 4 Kohlenstoffatomen im Alkylteil
- sowie Salze der Verbindungen der Formel (I) - gefunden.

Gesättigte oder ungesättigte Kohlenwasserstoffgruppierungen, wie Alkyl, Alkandiyl, Alkenyl oder Alkinyl, sind - auch in Verknüpfungen mit Heteroatomen, wie in Alkoxy - soweit möglich jeweils geradkettig oder verzweigt.

Gegebenenfalls substituierte Reste können einfach oder mehrfach substituiert sein, wobei bei Mehrfachsubstitution die Substituenten gleich oder verschieden sein können.

Bevorzugte Substituenten bzw. Bereiche der in den oben und nachstehend aufgeführten Formeln vorhandenen Reste werden im Folgenden definiert.
Q¹ steht bevorzugt für O (Sauerstoff) oder S (Schwefel).
Q² steht bevorzugt für O (Sauerstoff) oder S (Schwefel).
R¹ steht bevorzugt für jeweils gegebenenfalls durch Cyano, Fluor, Chlor, Methoxy oder Ethoxy substituiertes Methyl, Ethyl, n- oder i-Propyl, n-, i-, s-oder t-Butyl, für jeweils gegebenenfalls durch Cyano, Fluor oder Chlor substituiertes Propenyl, Butenyl, Propinyl oder Butinyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls durch Cyano, Fluor, Chlor, Methyl, Ethyl, n- oder i-Propyl, Trifluorethyl, Methoxy, Ethoxy, n- oder i-Propoxy, Difluormethoxy oder Trifluormethoxy substituiertes Phenyl, Phenylmethyl oder Phenylethyl, oder für jeweils gegebenenfalls durch Cyano, Fluor, Chlor, Brom, Methyl, Ethyl, n-oder i-Propyl, Methoxy, Ethoxy, n- oder i-Propoxy substituiertes Heterocyclyl oder Heterocyclylmethyl, wobei die Heterocyclylgruppe jeweils aus der Reihe Oxetanyl, Thietanyl, Furyl, Tetrahydrofuryl, Thienyl, Tetrahydrothienyl ausgewählt ist.
R² steht bevorzugt für Wasserstoff, Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n-oder i-Propoxy, Methoxycarbonyl, Ethoxycarbonyl, n- oder i-Propoxycarbonyl, Methylthio, Ethylthio, n- oder i-Propylthio, Methylsulfinyl, Ethylsulfmyl, Methylsulfonyl oder Ethylsulfonyl, oder für jeweils gegebenenfalls durch Cyano, Fluor oder Chlor substituiertes Propenyl, Butenyl, Propinyl, Butinyl, Propenyloxy, Butenyloxy, Propinyloxy oder Butinyloxy.
R³ steht bevorzugt, für jeweils durch Fluor oder Chlor substituiertes Methoxy, Ethoxy, n- oder i-Propoxy, n-, i-, s- oder t-Butoxy, n-, i-, s- oder t-Pentyloxy, oder neo-Pentyloxy.
R⁴ steht bevorzugt für Wasserstoff, Hydroxy, Amino, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor und/oder Brom substituiertes Ethenyl, Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylamino, Ethylamino, n- oder i-Propylamino, n-, i-, s- oder t-Butylamino, für Propenyloxy oder Butenyloxy, für Dimethylamino oder Diethylamino, für jeweils gegebenenfalls durch Fluor, Chlor, Methyl und/oder Ethyl substituiertes Cyclopropyl, Cyclobutyl, Cyclopentyl, Cyclohexyl, Cyclopropylamino, Cyclobutylamino, Cyclopentylamino, Cyclohexylamino, Cyclopropylmethyl, Cyclobutylmethyl, Cyclopentylmethyl oder Cyclohexylmethyl, oder für jeweils gegebenenfalls durch Fluor, Chlor, Methyl, Trifluormethyl und/oder Methoxy substituiertes Phenyl oder Benzyl.
Q¹ steht besonders bevorzugt für O (Sauerstoff) oder S (Schwefel).
Q² steht besonders bevorzugt für O (Sauerstoff) oder S (Schwefel).
R¹ steht besonders bevorzugt für jeweils gegebenenfalls durch Fluor, Chlor, Methoxy oder Ethoxy substituiertes Methyl, Ethyl, n- oder i-Propyl,

R² steht besonders bevorzugt für Fluor, Chlor, Brom oder für jeweils gegebenenfalls durch Fluor, Chlor, Methoxy oder Ethoxy substituiertes Methyl, Ethyl, n- oder i-Propyl.
R³ steht besonders bevorzugt für durch Fluor oder Chlor, substituiertes Methoxy, Ethoxy, n- oder i-Propoxy, n-, i-, s- oder t-Butoxy, n-, i-, s- oder t-Pentyloxy, neo-Pentyloxy.
R⁴ steht besonders bevorzugt für jeweils gegebenenfalls durch Fluor, Chlor, Methoxy oder Ethoxy substituiertes Methyl, Ethyl, n- oder i-Propyl, für jeweils gegebenenfalls durch Fluor oder Chlor substituiertes Ethenyl, Propenyl oder Propinyl, für jeweils gegebenenfalls durch Fluor, Chlor, Methoxy oder Ethoxy substituiertes Methoxy, Ethoxy, n- oder i-Propoxy, für Methylamino, oder für Cyclopropyl.

Gegenstand der Erfindung sind vorzugsweise auch die Natrium-, Kalium-, Lithium-, Magnesium-, Calcium-, Ammonium-, C₁-C₄-Alkyl-ammonium-, (wobei der Alkylrest gegebenenfalls durch Hydroxy substituiert ist), Di-(C₁-C₄-alkyl)-ammonium-, Tri-(C₁-C₄-alkyl)-ammonium-, Tetra-(C₁-C₄-akyl)-ammonium, Tri-(C₁-C₄-alkyl)-sulfonium-, C₅- oder C₆-Cycloalkyl-ammonium- und Di-(C₁-C₂-alkyl)-benzyl-ammonium-Salze sowie die Di-(C₁-C₂-alkyl)-pyridinyl-ammoniumSalze und die Pyrrolidiniumsalze von Verbindungen der Formel (I), in welcher Q¹, Q², R¹, R², R³ und R⁴ die oben vorzugsweise angegebenen Bedeutungen haben.

Eine ganz besonders bevorzugte Gruppe sind diejenigen Verbindungen der Formel (I), bei welchen
R¹ für Methyl steht und Q¹ und Q² sowie R², R³ und R⁴ die oben als besonders bevorzugt angegebenen Bedeutungen haben,
Eine weitere ganz besonders bevorzugte Gruppe sind diejenigen Verbindungen der Formel (I), bei welchen
R¹ für Ethyl steht und Q¹ und Q² sowie R², R³ und R⁴ die oben als besonders bevorzugt angegebenen Bedeutungen haben.

Eine weitere ganz besonders bevorzugte Gruppe sind diejenigen Verbindungen der Formel (I), bei welchen
R¹ für n-Propyl steht und Q¹ und Q² sowie R², R³ und R⁴ die oben als besonders bevorzugt angegebenen Bedeutungen haben.
Eine weitere ganz besonders bevorzugte Gruppe sind diejenigen Verbindungen der Formel (I), bei welchen
R¹ für i-Propyl steht und Q¹ und Q² sowie R², R³ und R⁴ die oben als besonders bevorzugt angegebenen Bedeutungen,
Eine weitere ganz besonders bevorzugte Gruppe sind diejenigen Verbindungen der Formel (I), bei welchen
Q¹ und Q² sowie R¹ und R² die oben als besonders bevorzugt angegebenen Bedeutungen haben und R³ und R⁴ zusammen für jeweils gegebenenfalls einfach oder zweifach durch Methyl substituiertes Trimethylen (Propan-1,3-diyl), 1-Oxa-trimethylen, 1-Thiatrimethylen, 1-Aza-trimethylen, Tetramethylen (Butan-1,4-diyl), 1-Oxa-tetramethylen, 1-Thia-tetramethylen, 1-Aza-tetramethylen, oder Pentamethylen (Pentan-1,5-diyl) stehen, wobei die Position 1 die über R³ markierte Bindungsstelle bedeutet.

Als weitere besonders hervorzuhebende Gruppe sei aufgezählt:

### Gruppe 1:

Verbindungen, bei denen R³ für durch Halogen substituiertes Alkoxy mit 1 bis 6 Kohlenstoffatomen steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Erfindungsgemäß bevorzugt sind diejenigen Verbindungen der Formel (I), bei welchen eine Kombination der vorstehend als bevorzugt aufgeführten Bedeutungen vorliegt.

Erfindungsgemäß besonders bevorzugt sind diejenigen Verbindungen der Formel (I), bei welchen eine Kombination der vorstehend als besonders bevorzugt aufgeführten Bedeutungen vorliegt.

Erfindungsgemäß ganz besonders bevorzugt sind diejenigen Verbindungen der Formel (I), bei welchen eine Kombination der vorstehend als ganz besonders bevorzugt aufgeführten Bedeutungen vorliegt.

Die neuen substituierten Thien-3-yl-sulfonylamino(thio)carbonyl-triazolin(thi)one der allgemeinen Formel (I) weisen interessante biologische Eigenschaften auf. Sie zeichnen sich insbesondere durch starke herbizide Wirksamkeit aus.

Man erhält die neuen substituierten Thien-3-yl-sulfonylamino(thio)carbonyl-triazolin(thi)one der allgemeinen Formel (I), wenn man
(a) substituierte Thiophen-3-sulfonamide der allgemeinen Formel (II) 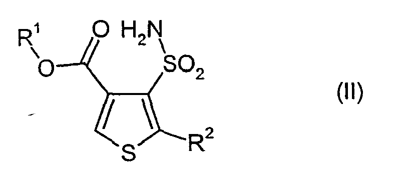 in welcher
   R¹ und R² die oben angegebene Bedeutung haben,
   mit substituierten Triazolin(thi)onen der allgemeinen Formel (III) 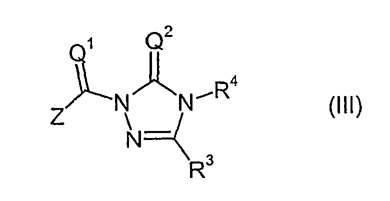 in welcher
   Q¹, Q², R³ und R⁴ die oben angegebene Bedeutung haben und
   Z für Halogen, Alkoxy, Aryloxy oder Arylalkoxy steht,
   gegebenenfalls in Gegenwart eines Reaktionshilfsmittels und gegebenenfalls in Gegenwart eines Verdünnungsmittels umsetzt,
   oder wenn man
(b) substituierte Thien-3-yl-sulfonyl-iso(thio)cyanate der allgemeinen Formel (IV) 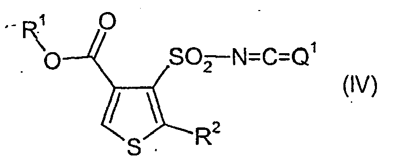 in welcher
   Q¹, R¹ und R² die oben angegebene Bedeutung haben,
   mit Triazolin(thi)onen der allgemeinen Formel (V) 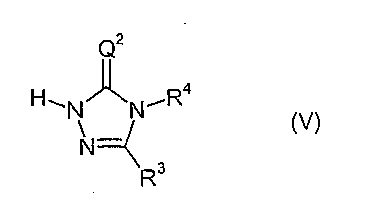 in welcher
   Q², R⁴ und R⁵ die oben angegebene Bedeutung haben,
   gegebenenfalls in Gegenwart eines Reaktionshilfsmittels und gegebenenfalls in Gegenwart eines Verdünnungsmittels umsetzt,
   oder wenn man
(c) substituierte Thiophen-3-sulfonsäurechloride der allgemeinen Formel (VI) 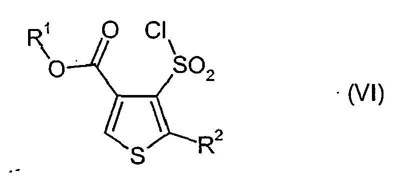 in welcher
   R¹ und R² die oben angegebene Bedeutung haben,
   mit Triazolin(thi)onen der allgemeinen Formel (V) 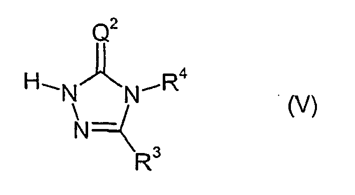 in welcher
   Q², R⁴ und R⁵ die oben angegebene Bedeutung haben,
   und Metall(thio)cyanaten der allgemeinen Formel (VII)

   M-Q¹-CN (VII)

   in welcher
   Q¹ die oben angegebene Bedeutung hat,
   gegebenenfalls in Gegenwart eines Reaktionshilfsmittels und gegebenenfalls in Gegenwart eines Verdünnungsmittels umsetzt,
   oder wenn man
(d) substituierte Thiophen-3-sulfonsäurechloride der allgemeinen Formel (VI) 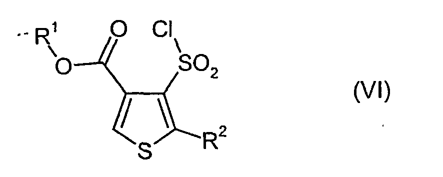 in welcher
   R¹ und R² die oben angegebene Bedeutung haben,
   mit Triazolin(thi)on-(thio)carboxamiden der allgemeinen Formel (VIII) 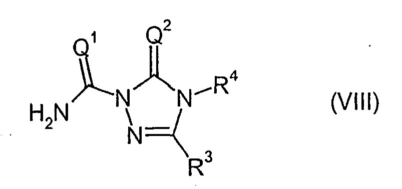 in welcher
   Q¹, Q², R³ und R⁴ die oben angegebene Bedeutung haben,
   gegebenenfalls in Gegenwart eines Reaktionshilfsmittels und gegebenenfalls in Gegenwart eines Verdünnungsmittels umsetzt,
   oder wenn man
(e) substituierte Thien-3-yl-sulfonylamino(thio)carbonylverbindungen der allgemeinen Formel (IX) 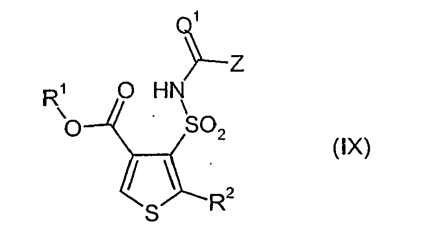
in welcher
Q¹, R¹ und R² die oben angegebene Bedeutung haben und
Z für Halogen, Alkoxy, Aryloxy oder Arylalkoxy steht,
mit Triazolin(thi)onen der allgemeinen Formel (V) 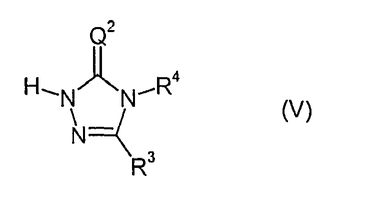 in welcher
Q², R⁴ und R⁵ die oben angegebene Bedeutung haben,
gegebenenfalls in Gegenwart eines Reaktionshilfsmittels und gegebenenfalls in Gegenwart eines Verdünnungsmittels umsetzt,
und gegebenenfalls die nach den Verfahren (a), (b), (c), (d) oder (e) erhaltenen Verbindungen der Formel (I) nach üblichem Methoden in Salze überführt.

Verwendet man beispielsweise 2-Brom-4-ethoxycarbonyl-thiophen-3-sulfonamid und 4,5-Dimethoxy-2-phenoxycarbonyl-2,4-dihydro-3H-1,2,4-triazol-3-on als Ausgangsstoffe, so kann der Reaktionsablauf beim erfindungsgemäßen Verfahren (a) durch das folgende Formelschema skizziert werden:

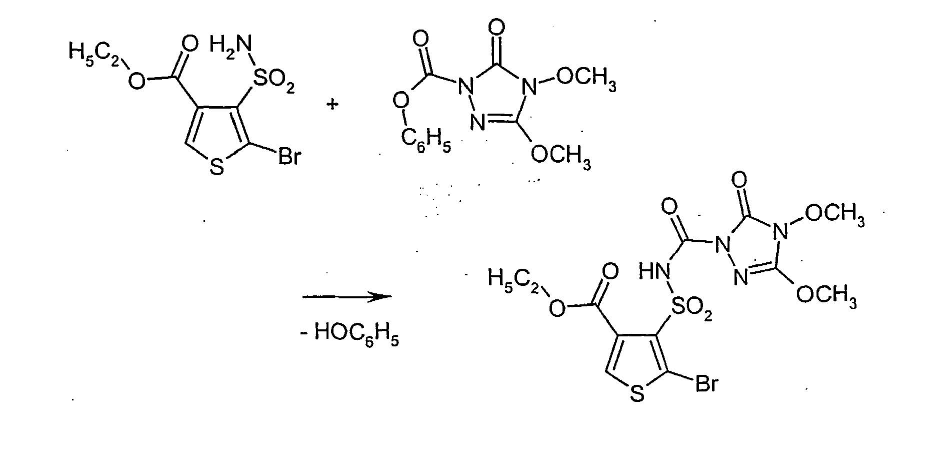

Verwendet man beispielsweise (2-Dichlormethyl-4-methoxycarbonyl-thien-3-yl-sulfonyl-isothiocyanat und 5-Ethoxy-4-methyl-2,4-dihydro-3H-1,2,4-triazol-3-on als Ausgangsstoffe, so kann der Reaktionsablauf beim erfindungsgemäßen Verfahren (b) durch das folgende Formelschema skizziert werden:

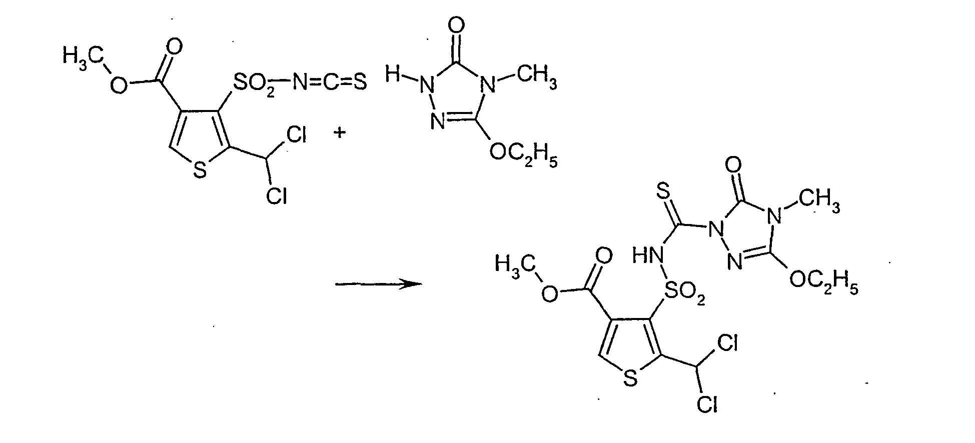

Verwendet man beispielsweise 4-Ethoxycarbonyl-2-ethyl-thiophen-3-sulfonsäurechlorid, 5-Ethyl-4-methoxy-2,4-dihydro-3H-1,2,4-triazol-3-thion und Kaliumcyanat als Ausgangsstoffe, so kann der Reaktionsablauf beim erfindungsgemäßen Verfahren (c) durch das folgende Formelschema skizziert werden:

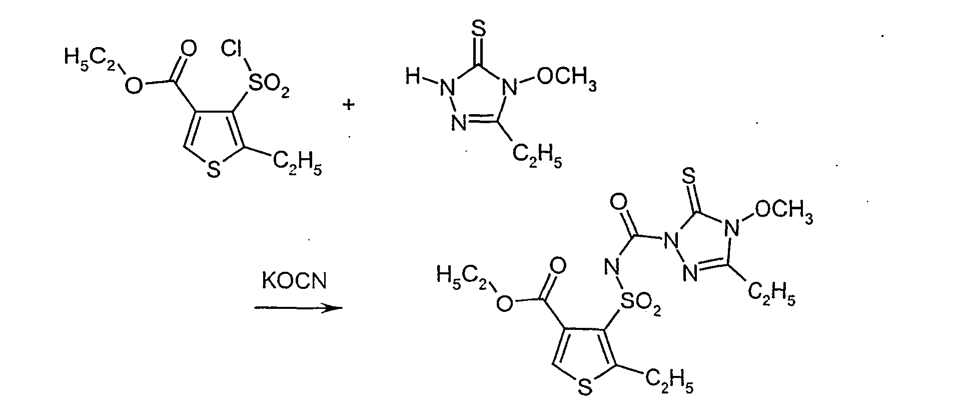

Verwendet man beispielsweise 4-Ethoxycarbonyl-2-trifluormethyl-thiophen-3-sulfonsäurechlorid und 4-Ethyl-5-methoxy-2,4-dihydro-3H-1,2,4-triazol-3-on-2-carboxamid als Ausgangsstoffe, so kann der Reaktionsablauf beim erfindungs-gemäßen Verfahren (d) durch das folgende Formelschema skizziert werden:

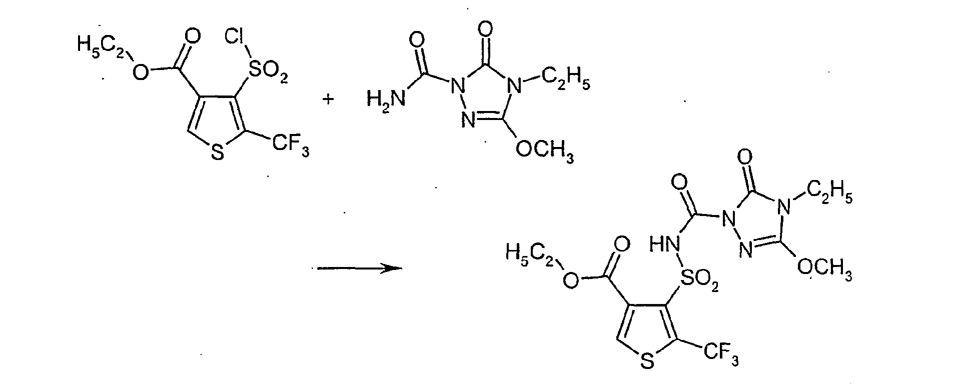

Verwendet man beispielsweise N-(2-Ethyl-4-i-propoxycarbonyl-thien-3-yl-sulfonyl)-O-methyl-urethan und 4,5-Dimethyl-2,4-dihydro-3H-1,2,4-triazol-3-on als Ausgangsstoffe, so kann der Reaktionsablauf beim erfindungsgemäßen Verfahren (e) durch das folgende Formelschema skizziert werden:

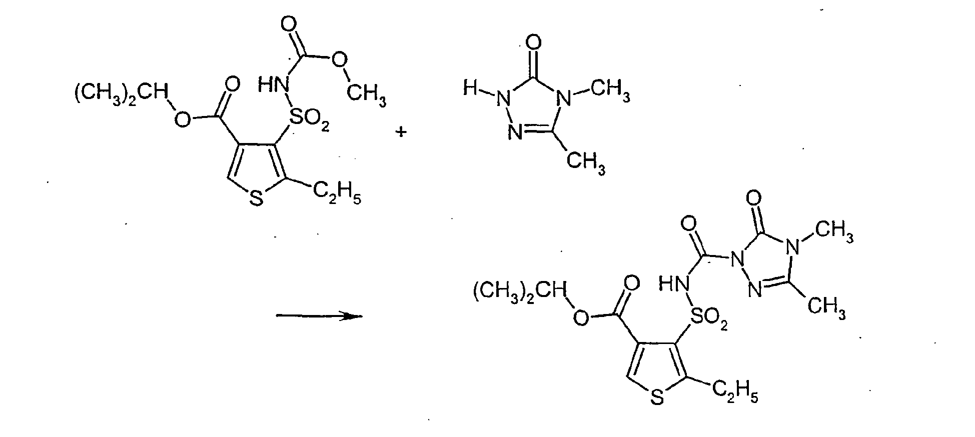

Die beim erfindungsgemäßen Verfahren (a) zur Herstellung von Verbindungen der allgemeinen Formel (I) als Ausgangsstoffe zu verwendenden substituierten Thiophen-3-sulfonamide sind durch die Formel (II) allgemein definiert. In der allgemeinen Formel (II) haben R¹ und R² vorzugsweise bzw. insbesondere diejenigen Bedeutungen, die bereits oben im Zusammenhang mit der Beschreibung der erfindungsgemäßen Verbindungen der allgemeinen Formel (I) vorzugsweise bzw. als insbesondere bevorzugt für R¹ und R² angegeben worden sind.

Die substituierten Thiophen-3-sulfonamide der allgemeinen Formel (II) sind bekannt und/oder können nach an sich bekannten Verfahren hergestellt werden (vgl. J. Org. Chem. 45 (1980), 617-620, WO-A-01/05788)

Man erhält die substituierten Thiophen-3-sulfonamide der allgemeinen Formel (II), wenn man substituierte Thiophen-3-sulfonsäurechloride der allgemeinen Formel (VI) 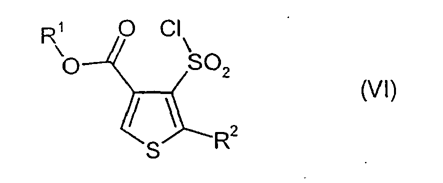 in welcher
R¹ und R² die oben angegebene Bedeutung haben,
mit Ammoniak oder mit Ammoniumsalzen, wie z.B. Ammoniumacetat oder Ammoniumcarbonat, gegebenenfalls in Gegenwart eines Verdünnungsmittels, wie z.B. Wasser oder Methylenchlorid, bei Temperaturen zwischen 0°C und 100°C umsetzt.

Die beim erfindungsgemäßen Verfahren (a) zur Herstellung von Verbindungen der allgemeinen Formel (I) weiter als Ausgangsstoffe zu verwendenden substituierten Triazolin(thi)one sind durch die Formel (III) allgemein definiert. In der allgemeinen Formel (III) haben Q¹, Q², R³ und R⁴ vorzugsweise bzw. insbesondere diejenigen Bedeutungen, die bereits oben im Zusammenhang mit der Beschreibung der erfindungsgemäßen Verbindungen der allgemeinen Formel (I) vorzugsweise bzw. als insbesondere bevorzugt für Q¹, Q², R³ und R⁴ angegeben worden sind.

Die Ausgangsstoffe der allgemeinen Formel (III) sind bekannt und/oder können nach an sich bekannten Verfahren hergestellt werden (vgl. EP-A-341 489, EP-A-422 469, EP-A-425 948, EP-A-431 291, EP-A-507 171, EP-A-534 266).

Die beim erfindungsgemäßen Verfahren (b) zur Herstellung von Verbindungen der allgemeinen Formel (I) als Ausgangsstoffe zu verwendenden substituierten Thien-3-yl-sulfonyl-iso(thio)cyanate sind durch die Formel (IV) allgemein definiert. In der allgemeinen Formel (IV) haben Q¹, R¹ und R² vorzugsweise bzw. insbesondere diejenigen Bedeutungen, die bereits oben im Zusammenhang mit der Beschreibung der erfindungsgemäßen Verbindungen der allgemeinen Formel (I) vorzugsweise bzw. als insbesondere bevorzugt für Q¹, R¹ und R² angegeben worden sind.

Die Ausgangsstoffe der allgemeinen Formel (IV) sind bekannt und/oder können nach an sich bekannten Verfahren hergestellt werden (vgl. US-A-47 01 535).

Die bei den erfindungsgemäßen Verfahren (b), (c) und (e) zur Herstellung von Verbindungen der allgemeinen Formel (I) als Ausgangsstoffe zu verwendenden Triazolin(thi)one sind durch die Formel (V) allgemein definiert. In der allgemeinen Formel (V) haben Q², R⁴ und R⁵ vorzugsweise bzw. insbesondere diejenigen Bedeutungen, die bereits oben im Zusammenhang mit der Beschreibung der erfindungsgemäßen Verbindungen der allgemeinen Formel (I) vorzugsweise bzw. als insbesondere bevorzugt für Q², R⁴ und R⁵ angegeben worden sind.

Die Ausgangsstoffe der allgemeinen Formel (V) sind bekannt und/oder können nach an sich bekannten Verfahren hergestellt werden (vgl. EP-A-341 489, EP-A-422 469, EP-A-425 948, EP-A-431 291, EP-A-507 171, EP-A-534 266).

Die bei den erfindungsgemäßen Verfahren (c) und (d) zur Herstellung von Verbindungen der allgemeinen Formel (I) als Ausgangsstoffe zu verwendenden substituierten Thiophen-3-sulfonsäurechloride sind durch die Formel (VI) allgemein definiert. In der allgemeinen Formel (VI) haben R¹ und R² vorzugsweise bzw. insbesondere diejenigen Bedeutungen, die bereits oben im Zusammenhang mit der Beschreibung der erfindungsgemäßen Verbindungen der allgemeinen Formel (I) vorzugsweise bzw. als insbesondere bevorzugt für R¹ und R² angegeben worden sind.

Die substituierten Thiophen-3-sulfonsäurechloride der allgemeinen Formel (VI) sind bekannt und/oder können nach an sich bekannten Verfahren hergestellt werden (vgl. J. Org. Chem. 45 (1980), 617-620, WO-A-01/05788)

Man erhält die substituierten Thiophen-3-sulfonsulfonsäurechloride der allgemeinen Formel (VI), wenn man 3-Amino-thiophen-4-carbonsäureester der allgemeinen Formel (X) 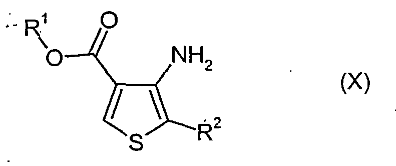 in welcher
R¹ und R² die oben angegebene Bedeutung haben,
- oder Säureaddukte von Verbindungen der Formel (X), wie z.B. die Hydrochloride -
mit einem Alkalimetallnitrit, wie z.B. Natriumnitrit, in Gegenwart von Salzsäure bei Temperaturen zwischen -10°C und +10°C umsetzt und die so erhaltene Diazoniumsalzlösung mit Schwefeldioxid in Gegenwart eines Verdünnungsmittels, wie z.B. Dichlormethan, 1,2-Dichlor-ethan oder Essigsäure, und in Gegenwart eines Katalysators, wie z.B. Kupfer(I)-chlorid und/oder Kupfer(II)-chlorid, bei Temperaturen zwischen -10°C und +50°C umsetzt.

Die Vorprodukte der allgemeinen Formel (X) sind bekannt und/oder können nach an sich bekannten Verfahren hergestellt werden (vgl. Austr. J. Chem. 48 (1995), 1907-1916; Herstellungsbeispiele).

Die beim erfindungsgemäßen Verfahren (d) zur Herstellung von Verbindungen der allgemeinen Formel (I) als Ausgangsstoffe zu verwendenden Triazolin(thi)on-(thio)-carboxamide sind durch die Formel (VIII) allgemein definiert. In der allgemeinen Formel (VIII) haben Q¹, Q², R³ und R⁴ vorzugsweise bzw. insbesondere diejenigen Bedeutungen, die bereits oben im Zusammenhang mit der Beschreibung der erfmdungsgemäßen Verbindungen der allgemeinen Formel (I) vorzugsweise bzw. als insbesondere bevorzugt für Q¹, Q², R³ und R⁴ angegeben worden sind.

Die Ausgangsstoffe der allgemeinen Formel (VIII) sind bekannt und/oder können nach an sich bekannten Verfahren hergestellt werden.

Die beim erfindungsgemäßen Verfahren (e) zur Herstellung von Verbindungen der allgemeinen Formel (I) als Ausgangsstoffe zu verwendenden substituierten Thien-3-yl-sulfonylamino(thio)carbonylverbindungen sind durch die Formel (IX) allgemein definiert. In der allgemeinen Formel (IX) haben Q¹, R¹ und R² vorzugsweise bzw. insbesondere diejenigen Bedeutungen, die bereits oben im Zusammenhang mit der Beschreibung der erfindungsgemäßen Verbindungen der allgemeinen Formel (I) vorzugsweise bzw. als insbesondere bevorzugt für Q¹, R¹ und R² angegeben worden sind.

Die Ausgangsstoffe der allgemeinen Formel (IX) sind bekannt und/oder können nach an sich bekannten Verfahren hergestellt werden.

Die erfindungsgemäßen Verfahren (a), (b), (c), (d) und (e) zur Herstellung der neuen Verbindungen der Formel (I) werden vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlormethan, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methylethyl-, Methyl-isopropyl- und Methyl-isobutyl-keton, Ester wie Essigssäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Als Reaktionshilfsmittel können bei den erfindungsgemäßen Verfahren (a), (b), (c), (d) und (e) alle üblicherweise für derartige Umsetzungen verwendbaren Säurebindemittel eingesetzt werden. Vorzugsweise in Frage kommen Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kalium-tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei den erfindungsgemäßen Verfahren (a), (b), (c), (d) und (e) in einem größeren Bereich variiert werden. Im Allgemeinen arbeitet man bei Temperaturen zwischen -20°C und +150°C, vorzugsweise bei Temperaturen zwischen 0°C und +100°C.

Die erfindungsgemäßen Verfahren (a), (b), (c), (d) und (e) werden im Allgemeinen unter Normaldruck durchgeführt. Es ist jedoch auch möglich, unter erhöhtem oder vermindertem Druck zu arbeiten.

Zur Durchführung der erfindungsgemäßen Verfahren (a), (b), (c), (d) und (e) werden die jeweils benötigten Ausgangsstoffe im Allgemeinen in angenähert äquimolaren Mengen eingesetzt. Es ist jedoch auch möglich, eine der jeweils eingesetzten Komponenten in einem größeren Überschuß zu verwenden. Die Reaktionen werden im Allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren (a), (b), (c), (d) und (e) jeweils nach üblichen Methoden (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Methylenchlorid, Aceton, tert-Butyl-methylether oder Toluol, und Zugabe einer geeigneten Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktioniert natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-FettalkoholEther, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise
Acetochlor, Acifluorfen (-sodium), Aclonifen, Alachlor, Alloxydim (-sodium), Ametryne, Amicarbazone, Amidochlor, Amidosulfuron, Anilofos, Asulam, Atrazine, Azafenidin, Azimsulfuron, Beflubutamid, Benazolin (-ethyl), Benfuresate, Bensulfuron (-methyl), Bentazon, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlomitrofen, Chlorsulfuron, Chlortoluron, Ciriidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flufenpyr, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Ketospiradox, Lactofen, Lenacil, Linuron, MCPA, Mecoprop, Mefenacet, Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Penoxysulam, Pentoxazone, Pethoxamid, Phenmedipham, Picolinafen, Piperophos, Pretilachlor, Primisulfuron (-methyl), Profluazol, Profoxydim, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, - Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Für die Mischungen kommen weiterhin bekannte Safener in Frage, beispielsweise
AD-67, BAS-145138, Benoxacor, Cloquintocet (-mexyl), Cyometrinil, 2,4-D, DKA-24, Dichlormid, Dymron, Fenclorim, Fenchlorazol (-ethyl), Flurazole, Fluxofenim, Furilazole, Isoxadifen (-ethyl), MCPA, Mecoprop (-P), Mefenpyr (-diethyl), MG-191, Oxabetrinil, PPG-1292, R-29148.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Förmulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden, gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit bestimmten Eigenschaften ("Traits"), die durch konventionelle Züchtung, durch Mutagenese, oder auch durch rekombinante DNA-Techniken erhalten worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfmdungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel - auch in Kombination mit anderen agrochemischen Wirkstoffen, besseres Wachstum der Kulturpflanzen, erhöhte Toleranz der Kulturpflanzen gegenüber hohen oder niedrigen Temperaturen, erhöhte Toleranz der Kulturpflanzen gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch- erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinothricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinothricin, z.B. Raps), IMI® (Toleranz gegen Imidazolinone) und STS® (Toleranz gegen Sulfonylharnstoffe z.B. Mais) vertrieben werden. Als Herbizidresistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden, wobei zusätzlich zu der guten Bekämpfung der Unkrautpflanzen die oben genannten synergistischen Effekte mit den transgenen Pflanzen oder Pflanzensorten auftreten. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den nachfolgenden Beispielen hervor.

### Herstellungsbeispiele:

### Beispiel 1 (Referenz-Beispiel)

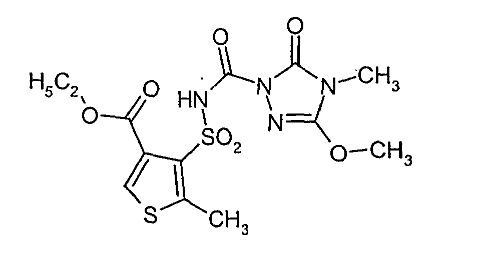

0,45 g (2,19 mMol) 5-Methoxy-4-methyl-2-phenoxycarbonyl-2,4-dihydro-3H-1,2,4-triazol-3-on werden in 50 ml Acetonitril gelöst und bei Raumtemperatur (ca. 20°C) unter Rühren portionsweise mit 0,60 g (2,41 mMol) 4-Ethoxycarbonyl-2-methyl-thiophen-3-sulfonamid und mit 0,37 g (2,41 mMol) 1,8-Diazabicyclo-[5.4.0]-undec-7-en (DBU) versetzt. Die Reaktionsmischung wird 12 Stunden bei Raumtemperatur gerührt und anschließend unter vermindertem Druck eingeengt. Der Rückstand wird in Methylenchlorid aufgenommen und nacheinander mit 1N-Salzsäure und mit Wasser gewaschen, mit Natriumsulfat getrocknet und filtriert. Das Filtrat wird unter vermindertem Druck eingeengt, der Rückstand mit Isopropanol digeriert und das kristallin angefallene Produkt durch Absaugen isoliert.

Man erhält 0,60 g (68 % der Theorie) 4-[[[(3-Methoxy-4-methyl-5-oxo-1H-1,2,4-triazol-1-yl)-carbonyl]-amino]-sulfonyl]-5-methyl-3-thiophen-carbonsäure-ethylester als hellgelben Feststoff vom Schmelzpunkt 176°C.

Das Natriumsalz der gemäß Beispiel 1 herzustellenden Verbindung kann beispielsweise wie folgt hergestellt werden:
1,0 g (2,5 mMol) 4-[[[(3-Methoxy-4-methyl-5-oxo-1H-1,2,4-triazol-1-yl)-carbonyl]-amino]-sulfonyl]-5-methyl-3-thiophen-carbonsäure-ethylester werden in 25 ml Methylenchlorid aufgenommen und mit 0,10 g (2,5 mMol) Natriumhydroxid (Micropills) versetzt. Die Mischung wird 15 Stunden bei Raumtemperatur (ca. 20°C) gerührt. Dann wird das kristalline Produkt durch Absaugen isoliert.
Man erhält 1,0 g 4-[[[(3-Methoxy-4-methyl-5-oxo-1H-1,2,4-triazol-1-yl)-carbonyl]-amino]-sulfonyl]-5-methyl-3-thiophen-carbonsäure-ethylester-Natriumsalz vom Schmelzpunkt 220°C.

Analog zu Beispiel 1 sowie entsprechend der allgemeinen Beschreibung des erfindungsgemäßen Herstellungsverfahrens können beispielsweise auch die in der nachstehenden Tabelle 1 aufgeführten Verbindungen der allgemeinen Formel (I) hergestellt werden.

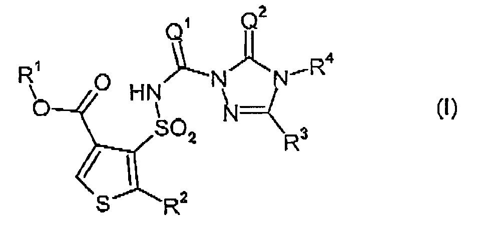

**Tabelle 1: Beispiele für die Verbindungen der Formel (I)**

| **Bsp.- Nr.** | **Q¹** | **Q²** | **R¹** | **R²** | **R³** | **R⁴** | **Schmelzpunkt (°C)** |
|---|---|---|---|---|---|---|---|
| 9 | ○ | ○ | CH₃ | CH₃ | 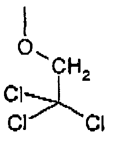 | CH₃ | 170 |
| 41 | ○ | ○ | CH₃ | CH₃ | OCH₂CF₃ | CH₃ | 197 |
| 43 | ○ | ○ | CH₃ | CH₃ | OCH₂CF₃ | 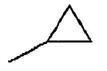 | 117 |
| 117 | ○ | ○ | C₃H₇-i | CH₃ | OCH₂CF₃ | CH₃ | 188 |
| 119 | ○ | ○ | C₃H₇-i | CH₃ | OCH₂CF₃ | 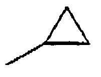 | 255 |
| 164 | ○ | ○ | C₂H₅ | CH₃ | OCH₂CF₃ | CH₃ | 175 |
| 166 | ○ | ○ | C₂H₅ | CH₃ | OCH₂CF₃ | 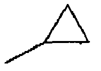 | 147 |
| 195 | ○ | ○ | C₃H₇-n | CH₃ | OCH₂CF₃ | CH₃ | 140 |
| 197 | ○ | ○ | C₃H₇-n | CH₃ | OCH₂CF₃ | 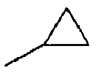 | 122 |
| 217 | ○ | ○ | CH₃ | C₂H₅ | OCH₂CF₃ | CH₃ | 163 |
| 219 | ○ | ○ | CH₃ | C₂H₅ | OCH₂CF₃ | 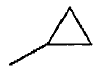 | 161 |

### Anwendungsbeispiele:

Bei den Anwendungsbeispielen werden die folgenden vorbekannten Verbindungen (alle bekannt aus WO-A-01/05788) zum Vergleich herangezogen: 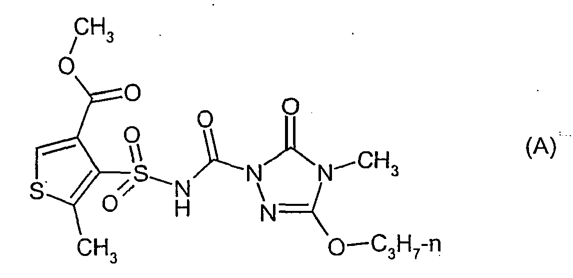 4-[[[(4,5-Dihydro-4-methyl-5-oxo-3-n-propoxy-1H-1,2,4-triazol-1-yl)-carbonyl]-amino]-sulfonyl]-5-methyl-3-thiophencarbonsäure-methylester (A) 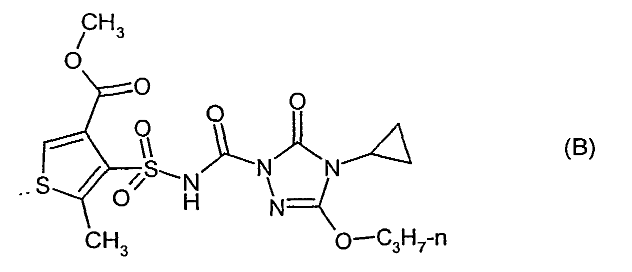 4-[[[(4-Cyclopropyl-4,5-dihydro-5-oxo-3-n-propoxy-1H-1,2,4-triazol-1-yl)-carbonyl]-amino]-sulfonyl]-5-methyl-3-thiophencarbonsäure-methylester (B) 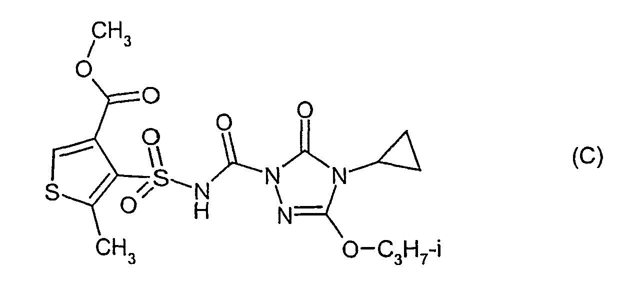 4-[[[(4-Cyclopropyl-4,5-dihydro-5-oxo-3-i-propoxy-1H-1,2,4-triazol-1-yl)-carbonyl]-amino]-sulfonyl]-5-methyl-3-thiophencarbonsäure-methylester (C) 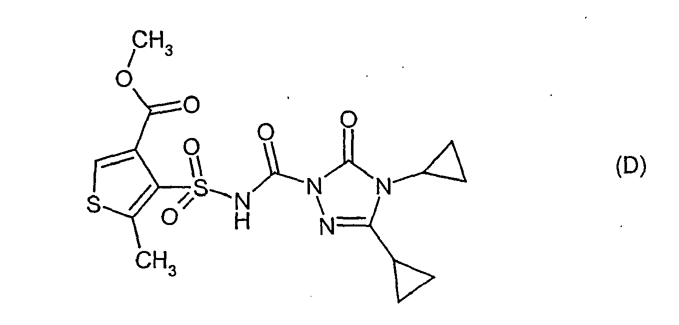 4-[[[(3,4-Dicyclopropyl-4,5-dihydro-5-oxo-1H-1,2,4-triazol-1-yl)-carbonyl]-amino]-sulfonyl]-5-methyl-3-thiophencarbonsäure-methylester (D) 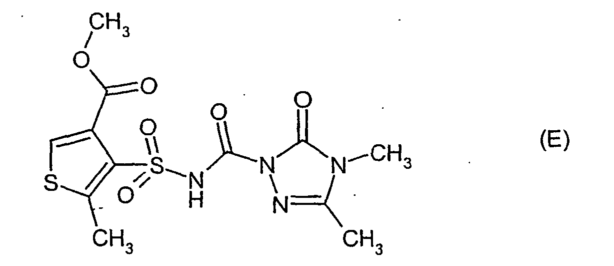 4-[[[(4,5-Dihydro-3,4-dimethyl-5-oxo-1H-1,2,4-triazol-1-yl)-carbonyl]-amino]-sulfonyl]-5-methyl-3-thiophencarbonsäure-methylester (E) 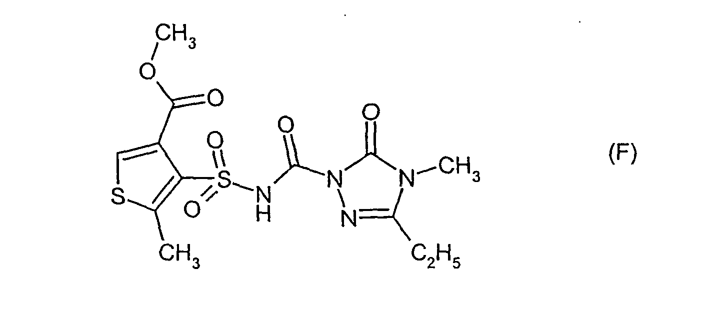 4-[[[(4,5-Dihydro-3-ethyl-4-methyl-5-oxo-1H-1,2,4-triazol-1-yl)-carbonyl]-amino]-sulfonyl]-5-methyl-3-thiophencarbonsäure-methylester (F) 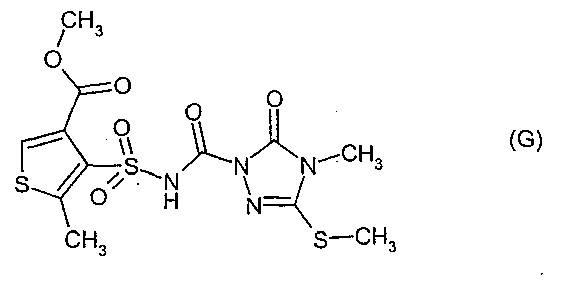 4-[[[(4,5-Dihydro-4-methyl-3-methylthio-5-oxo-1H-1,2,4-triazol-1-yl)-carbonyl]-amino]-sulfonyl]-5-methyl-3-thiophencarbonsäure-methylester (G)

### Beispiel A

### Post-emergence-Test

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, dass die jeweils gewünschten Wirkstoffmengen pro Flächeneinheit ausgebracht werden. Die Konzentration der Spritzbrühe wird so gewählt, dass in 1000 I Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigen beispielsweise die Verbindungen gemäß Herstellungsbeispiel 1, erheblich stärkere Wirkung gegen Unkräuter und weitgehend bessere Verträglichkeit gegenüber Kulturpflanzen wie z.B. Mais, Raps und Weizen, als die bekannten Verbindungen (A) und (B).

### Beispiel B

### Pre-emergence-Test

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach 24 Stunden wird der Boden so mit der Wirkstoffzubereitung besprüht, dass die jeweils gewünschte Wirkstoffmenge pro Flächeneinheit ausgebracht wird. Die Wirkstoffkonzentration in der Spritzbrühe wird so gewählt, dass in 1000 Liter Wasser pro Hektar die jeweils gewünschte Wirkstoffmenge ausgebracht wird.

Nach drei Wochen wird der Schädigungsgrad der Pflanzen bonitiert in % Schädigung im Vergleich zur Entwicklung der unbehandelten Kontrolle. Es bedeuten:

| | |
|---|---|
| 0 % = | keine Wirkung (wie unbehandelte Kontrolle) |
| 100 % = | totale Vernichtung |

In diesem Test zeigen beispielsweise die Verbindungen gemäß Herstellungsbeispiel 41 und 43, bei weitgehend guter Verträglichkeit gegenüber Kulturpflanzen, wie z.B. Mais, Soja und Weizen, erheblich stärkere Wirkung gegen Unkräuter als die bekannten Verbindungen (A), (B), (C), (D), (E), (F) und (G).

**Tabelle A1: Post-emergence-Test/Gewächshaus**

| Wirkstoff gemäß Herstellungsbeispielnr. | Aufwandmenge (g ai/ha) | Weizen | Mais | Echino-chloa | Lolium | Datura | Polygonum | Viola | Xanthium |
|---|---|---|---|---|---|---|---|---|---|
| (B) | 15 | 30 | 90 | - | 60 | 80 | 70 | - | 50 |
| (A) | 15 | - | 30 | 60 | 30 | 50 | 50 | - | 70 |
| (1) | 15 | 0 | 0 | 90 | 90 | 95 | 95 | 95 | 95 |

**Tabelle B1: Pre-emergence-Test/Gewächshaus**

| Wirkstoff gemäß Herstellungsbeispielnr. | Aufwandmenge (g ai/ha) | Weizen | Mais | Bronius | Lolium | Amaranthus | Matricaria | Solanum | Stellaria | Xanthium |
|---|---|---|---|---|---|---|---|---|---|---|
| (G) | 15 | 0 | 0 | - | 60 | 40 | 70 | 80 | 80 | 40 |
| (F) | 15 | 0 | 0 | 70 | 40 | 40 | 20 | 80 | 70 | 0 |
| (E) | 15 | 0 | 0 | 20 | 0 | 10 | 20 | 70 | 40 | 0 |
| (D) | 15 | 0 | 20 | 10 | 20 | 80 | 10 | 80 | 60 | 0 |
| (C) | 15 | 0 | 0 | 70 | 50 | 80 | 80 | - | 80 | 0 |
| (B) | 15 | 0 | 0 | 40 | 20 | 80 | 40 | 80 | 60 | 0 |
| (A) | 15 | 0 | 0 | 60 | 0 | - | 50 | 80 | 80 | 0 |
| (1) | 15 | 0 | 0 | 95 | 90 | 100 | 100 | 100 | 100 | 95 |

**Tabelle B2: Pre-emergence-Test/Gewächshaus**

| Wirkstoff gemäß Herstellungsbeispielnr. | Aufwandmenge (g ai/ha) | Weizen | Mais | Alopecurus | Selaria | Amaranthus | Chenopodium | Solanum | Stellaria |
|---|---|---|---|---|---|---|---|---|---|
| (G) | 15 | 0 | 0 | - | 70 | 40 | 50 | 80 | 80 |
| (F) | 15 | 0 | 0 | 70 | 30 | 40 | 0 | 80 | 70 |
| (E) | 15 | 0 | 0 | 20 | 20 | 10 | 0 | 70 | 40 |
| (D) | 15 | 0 | 20 | 0 | 10 | 80 | 0 | 80 | 60 |
| (C) | 15 | 0 | 0 | 0 | 50 | 80 | 40 | - | 80 |
| (B) | 15 | 0 | 0 | 20 | 40 | 80 | 40 | 80 | 60 |
| (A) | 15 | 0 | 0 | 60 | 0 | - | 50 | 80 | 80 |
| (41) | 15 | 0 | 0 | 100 | 95 | 100 | 100 | 100 | 100 |

**Tabelle B3: Pre-emergence-Test/Gewächshaus**

| Wirkstoff gemäß Herstellungsbeispielnr. | Aufwandmenge (g ai/ha) | Avena fatua | Lolium | Setaria | Amaranthus | Matricaria | Polygonum | Stellaria |
|---|---|---|---|---|---|---|---|---|
| (G) | 15 | 50 | 60 | 70 | 40 | 70 | 70 | 80 |
| (F) | 15 | 60 | 40 | 30 | 40 | 20 | 50 | 70 |
| (E) | 15 | 0 | 0 | 20 | 10 | 20 | 20 | 40 |
| (D) | 15 | 70 | 20 | 10 | 80 | 10 | 10 | 60 |
| (C) | 15 | 50 | 50 | 50 | 80 | 80 | 60 | 80 |
| (B) | 15 | 10 | 20 | 40 | 80 | 40 | 50 | 60 |
| (A) | 15 | 0 | 0 | 0 | - | 50 | 0 | 80 |
| (43) | 15 | - | 95 | 95 | 100 | 100 | 100 | 100 |

**Tabelle A8: Post-emergence-Test/Gewächshaus**

| Wirkstoff gemäß Herstellungsbeispielnr. | Aufwandmenge (g ai/ha) | Avena fatua | Bromus | Digitaria | Echinochloa | Lolium | Setaria |
|---|---|---|---|---|---|---|---|
| (A) | 15 | 30 | 50 | 10 | 60 | 30 | 30 |
| (39) | 15 | 90 | 95 | 95 | 100 | 95 | 95 |
| (45) | 15 | 90 | 90 | 100 | 100 | 95 | 90 |

**Tabelle A9: Post-emergence-Test/Gewächshaus**

| Wirkstoff gemäß Herstellungsbeispielnr. | Aufwandmenge (g ai/ha) | Raps | Alopecurus | Echinochloa | Lolium | Chenopodium |
|---|---|---|---|---|---|---|
| (A) | 15 | 95 | 70 | 60 | 30 | 80 |
| (42) | 15 | 0 | 90 | 90 | 90 | 95 |

**Tabelle A10: Post-emergence-Test/Gewächshaus**

| Wirkstoff gemäß Herstellungsbeispielnr. | Aufwandmenge (g ai/ha) | Avena fatua | Digitaria | Echinochloa | Lolium | Setaria | Viola | Xanthium |
|---|---|---|---|---|---|---|---|---|
| (A) | 15 | 30 | 10 | 60 | 30 | 30 | 30 | 70 |
| (47) | 15 | 95 | 90 | 95 | 100 | 90 | 100 | - |
| (48) | 15 | 90 | 90 | 95 | 100 | 80 | 95 | 90 |

**Tabelle A11: Post-emergence-Test/Gewächshaus**

| Wirkstoff gemäß Herstellungsbeispielnr. | Aufwandmenge (g ai/ha) | Avena fatua | Bromus | Digitaria | Lolium | Setaria | Xanthium |
|---|---|---|---|---|---|---|---|
| (B) | 15 | 50 | 50 | 20 | 60 | 60 | 50 |
| (39) | 15 | 90 | 95 | 95 | 95 | 95 | - |
| (45) | 15 | 90 | 90 | 100 | 95 | 90 | - |
| (47) | 15 | 95 | 80 | 90 | 100 | 90 | 80 |

**Tabelle A12: Post-emergence-Test/Gewächshaus**

| Wirkstoff gemäß Herstellungsbeispielnr. | Aufwandmenge (g ai/ha) | Alopecurus | Avena fatua | Digitaria | Lolium | Xanthium |
|---|---|---|---|---|---|---|
| (B) | 15. | 0 | 50 | 20 | 60 | 50 |
| (46) | 15 | 80 | 80 | 90 | 95 | - |
| (48) | 15 | - | 90 | 90 | 100 | 90 |

**Tabelle B1: Pre-emergence-Test/Gewächshaus**

| Wirkstoff gemäß Herstellungsbeispielnr. | Aufwandmenge (g ai/ha) | Weizen | Mais | Bromus | Lolium | Amaranthus | Matricaria | Solanum | Stellaria | Xanthium |
|---|---|---|---|---|---|---|---|---|---|---|
| (G) | 15 | 0 | 0 | - | 60 | 40 | 70 | 80 | 80 | 40 |
| (F) | 15 | 0 | 0 | 70 | 40 | 40 | 20 | 80 | 70 | 0 |
| (E) | 15 | 0 | 0 | 20 | 0 | 10 | 20 | 70 | 40 | 0 |
| (D) | 15 | 0 | 20 | 10 | 20 | 80 | 10 | 80 | 60 | 0 |
| (C) | 15 | 0 | 0 | 70 | 50 | 80 | 80 | - | 80 | 0 |
| (B) | 15 | 0 | 0 | 40 | 20 | 80 | 40 | 80 | 60 | 0 |
| (A) | 15 | 0 | 0 | 60 | 0 | - | 50 | 80 | 80 | 0 |
| (56) | 15 | 0 | 0 | 95 | 95 | 100 | 100 | 100 | 100 | 95 |
| (1) | 15 | 0 | 0 | 95 | 90 | 100 | 100 | 100 | 100 | 95 |

**Tabelle B2: Pre-emergence-Test/Gewächshaus**

| Wirkstoff gemäß Herstellungsbeispielnr. | Aufwandmenge (g ai/ha) | Weizen | Mais | Soja | Alopecurus | Lolium | Amaranthus | Matricaria | Solanum | Stellaria |
|---|---|---|---|---|---|---|---|---|---|---|
| (G) | 15 | 0 | 0 | 50 | - | 60 | 40 | 70 | 80 | 80 |
| (F) | 15 | 0 | 0 | 10 | 70 | 40 | 40 | 20 | 80 | 70 |
| (E) | 15 | 0 | 0 | 0 | 20 | 0 | 10 | 20 | 70 | 40 |
| (D) | 15 | 0 | 20 | 0 | 0 | 20 | 80 | 10 | 80 | 60 |
| (C) | 15 | 0 | 0 | 0 | 0 | 50 | 80 | 80 | - | 80 |
| (B) | 15 | 0 | 0 | 0 | 20 | 20 | 80 | 40 | 80 | 60 |
| (A) | 15 | 0 | 0 | 0 | 60 | 0 | - | 50 | 80 | 80 |
| (20) | 15 | 0 | 0 | 0 | 90 | 95 | 95 | 100 | 100 | 100 |

**Tabelle B3: Pre-emergence-Test/Gewächshaus**

| Wirkstoff gemäß Herstellungsbeispielnr. | Aufwandmenge (g ai/ha) | Weizen | Mais | Alopecurus | Bromus | Lolium | Polygonum | Solanum | Stellaria | Xanthium |
|---|---|---|---|---|---|---|---|---|---|---|
| (G) | 15 | 0 | 0 | - | - | 60 | - | 80 | 80 | 40 |
| (F) | 15 | 0 | 0 | 70 | 70 | 40 | 50 | 80 | 70 | 0 |
| (E) | 15 | 0 | 0 | 20 | 20 | 0 | 20 | 70 | 40 | 0 |
| (D) | 15 | 0 | 20 | 0 | 10 | 20 | 10 | 80 | 60 | 0 |
| (C) | 15 | 0 | 0 | 0 | 70 | 50 | 60 | - | 80 | 0 |
| (B) | 15 | 0 | 0 | 20 | 40 | 20 | 50 | 80 | 60 | 0 |
| (A) | 15 | 0 | 0 | 60 | 60 | 0 | 0 | 80 | 80 | 0 |
| (55) | 15 | 0 | 0 | 95 | 95 | 90 | 95 | 100 | 100 | 95 |
| (22) | 15 | 0 | 0 | 95 | 100 | 95 | 95 | 100 | 100 | - |
| (15) | 15 | 0 | 0 | 100 | 90 | 95 | 90 | 95 | 100 | 95 |

**Tabelle B4: Pre-emergence- Test/Gewächshaus**

| Wirkstoff gemäß Herstellungsbeispielnr. | Aufwandmenge (g ai/ha) | Weizen | Mais | Soja | Alopecurus | Setaria | Amaranthus | Matricaria | Stellaria |
|---|---|---|---|---|---|---|---|---|---|
| (G) | 15 | 0 | 0 | 50 | - | 70 | 40 | 70 | 80 |
| (F) | 15 | 0 | 0 | 10 | 70 | 30 | 40 | 20 | 70 |
| (E) | 15 | 0 | 0 | 0 | 20 | 20 | 10 | 20 | 40 |
| (D) | 15 | 0 | 20 | 0 | 0 | 10 | 80 | 10 | 60 |
| (C) | 15 | 0 | 0 | 0 | 0 | 50 | 80 | 80 | 80 |
| (B) | 15 | 0 | 0 | 0 | 20 | 40 | 80 | 40 | 60 |
| (A) | 15 | 0 | 0 | 0 | 60 | 0 | - | 50 | 80 |
| (18) | 15 | 0 | 0 | 0 | 95 | 95 | 95 | 100 | 95 |

**Tabelle B5: Pre-emergence-Test/Gewächshaus**

| Wirkstoff gemäß Herstellungsbeispielnr. | Aufwandmenge (g ai/ha) | Mais | Soja | Lolium | Setaria | Amaranthus | Chenopodium | Solanum | Stellaria |
|---|---|---|---|---|---|---|---|---|---|
| (G) | 15 | 0 | 50 | 60 | 70 | 40 | 50 | 80 | 80 |
| (F) | 15 | 0 | 10 | 40 | 30 | 40 | 0 | 80 | 70 |
| (E) | 15 | 0 | 0 | 0 | 20 | 10 | 0 | 70 | 40 |
| (D) | 15 | 20 | 0 | 20 | 10 | 80 | 0 | 80 | 60 |
| (C) | 15 | 0 | 0 | 50 | 50 | 80 | 40 | - | 80 |
| (B) | 15 | 0 | 0 | 20 | 40 | 80 | 40 | 80 | 60 |
| (A) | 15 | 0 | 0 | 0 | 0 | - | 50 | 80 | 80 |
| (38) | 15 | 0 | 0 | 80 | 95 | 100 | 95 | 100 | 100 |

**Tabelle B6: Pre-emergence-Test/Gewächshaus**

| Wirkstoff gemäß Herstellungsbeispielnr. | Aufwandmenge (g ai/ha) | Weizen | Mais | Alopecurus | Setaria | Amaranthus | Chenopodium | Solanum | Stellaria |
|---|---|---|---|---|---|---|---|---|---|
| (G) | 15 | 0 | 0 | - | 70 | 40 | 50 | 80 | 80 |
| (F) | 15 | 0 | 0 | 70 | 30 | 40 | 0 | 80 | 70 |
| (E) | 15 | 0 | 0 | 20 | 20 | 10 | 0 | 70 | 40 |
| (D) | 15 | 0 | 20 | 0 | 10 | 80 | 0 | 80 | 60 |
| (C) | 15 | 0 | 0 | 0 | 50 | 80 | 40 | - | 80 |
| (B) | 15 | 0 | 0 | 20 | 40 | 80 | 40 | 80 | 60 |
| (A) | 15 | 0 | 0 | 60 | 0 | - | 50 | 80 | 80 |
| (41) | 15 | 0 | 0 | 100 | 95 | 100 | 100 | 100 | 100 |

**Tabelle B7: Pre-emergence-Test/Gewächshaus**

| Wirkstoff gemäß Herstellungsbeispielnr. | Aufwandmenge (g ai/ha) | Weizen | Digitaria | Lolium | Setaria | Amaranthus | Solanum | Stellaria |
|---|---|---|---|---|---|---|---|---|
| (G) | 15 | 0 | 50 | 60 | 70 | 40 | 80 | 80 |
| (F) | 15 | 0 | 60 | 40 | 30 | 40 | 80 | 70 |
| (E) | 15 | 0 | 0 | 0 | 20 | 10 | 70 | 40 |
| (D) | 15 | 0 | 20 | 20 | 10 | 80 | 80 | 60 |
| (C) | 15 | 0 | 20 | 50 | 50 | 80 | - | 80 |
| (B) | 15 | 0 | 0 | 20 | 40 | 80 | 80 | 60 |
| (A) | 15 | 0 | 0 | 0 | 0 | - | 80 | 80 |
| (39) | 15 | 0 | 100 | 95 | 100 | 100 | 95 | 100 |

**Tabelle B8: Pre-emergence-Test/Gewächshaus**

| Wirkstoff gemäß Herstellungsbeispielnr. | Aufwandmenge (g ai/ha) | Weizen | Mais | Soja | Alopecurus | Lolium | Matricaria | Stellaria |
|---|---|---|---|---|---|---|---|---|
| (G) | 15 | 0 | 0 | 50 | - | 60 | 70 | 80 |
| (F) | 15 | 0 | 0 | 10 | 70 | 40 | 20 | 70 |
| (E) | 15 | 0 | 0 | 0 | 20 | 0 | 20 | 40 |
| (D) | 15 | 0 | 20 | 0 | 0 | 20 | 10 | 60 |
| (C) | 15 | 0 | 0 | 0 | 0 | 50 | 80 | 80 |
| (B) | 15 | 0 | 0 | 0 | 20 | 20 | 40 | 60 |
| (A) | 15 | 0 | 0 | 0 | 60 | 0 | 50 | 80 |
| (46) | 15 | 50 | 0 | 0 | 95 | 90 | 100 | 100 |

**Tabelle B9: Pre-emergence-Test/Gewächshaus**

| Wirkstoff gemäß Herstellungsbeispielnr. | Aufwandmenge (g ai/ha) | Avena fatua | Lolium | Setaria | Amaranthus | Matricaria | Polygonum | Stellaria |
|---|---|---|---|---|---|---|---|---|
| (G) | 15 | 50 | 60 | 70 | 40 | 70 | 70 | 80 |
| (F) | 15 | 60 | 40 | 30 | 40 | 20 | 50 | 70 |
| (E) | 15 | 0 | 0 | 20 | 10 | 20 | 20 | 40 |
| (D) | 15 | 70 | 20 | 10 | 80 | 10 | 10 | 60 |
| (C) | 15 | 50 | 50 | 50 | 80 | 80 | 60 | 80 |
| (B) | 15 | 10 | 20 | 40 | 80 | 40 | 50 | 60 |
| (A) | 15 | 0 | 0 | 0 | - | 50 | 0 | 80 |
| (43) | 15 | - | 95 | 95 | 100 | 100 | 100 | 100 |
| (45) | 15 | 95 | 100 | 100 | 100 | 100 | 95 | 100 |
| (47) | 15 | 95 | 80 | 95 | 100 | 100 | 90 | 100 |
| (48) | 15 | 95 | 95 | - | 100 | 95 | 95 | 100 |

**Tabelle B10: Pre-emergence-Test/Gewächshaus**

| Wirkstoff gemäß Herstellungsbeispielnr. | Aufwandmenge (g ai/ha) | Avena fatua | Bromus | Lolium | Setaria | Amaranthus | Galium | Stellaria |
|---|---|---|---|---|---|---|---|---|
| (G) | 15 | 50 | - | 60 | 70 | 40 | 30 | 80 |
| (F) | 15 | 60 | 70 | 40 | 30 | 40 | 10 | 70 |
| (E) | 15 | 0 | 20 | 0 | 20 | 10 | 0 | 40 |
| (D) | 15 | 70 | 10 | 20 | 10 | 80 | 0 | 60 |
| (C) | 15 | 50 | 70 | 50 | 50 | 80 | 70 | 80 |
| (B) | 15 | 10 | 40 | 20 | 40 | 80 | 20 | 60 |
| (A) | 15 | 0 | 60 | 0 | 0 | - | 10 | 80 |
| (42) | 15 | 90 | 95 | 95 | 95 | 100 | 100 | 100 |

## Patentansprüche

1. Verbindungen der allgemeinen Formel (I) 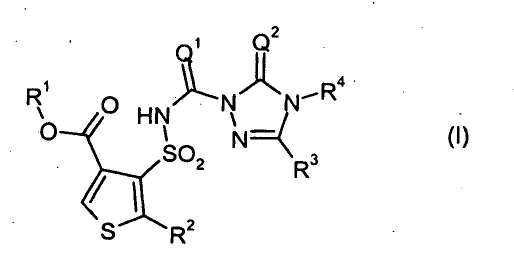 in welcher
Q¹ für O (Sauerstoff) oder S (Schwefel) steht,
Q² für O (Sauerstoff) oder S (Schwefel) steht,
R¹ für gegebenenfalls durch Cyano, Halogen oder C₁-C₄-Alkoxy substituiertes Alkyl mit 1 bis 6 Kohlenstoffatomen, für jeweils gegebenenfalls durch Cyano oder Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil, für jeweils gegebenenfalls durch Nitro, Cyano, Halogen, C₁-C₄-Alkyl oder C₁-C₄-Alkoxy substituiertes Aryl oder Arylalkyl mit jeweils 6 oder 10 Kohlenstoffatomen in der Arylgruppe und gegebenenfalls 1 bis 4 Kohlenstoffatomen im Alkylteil, oder für jeweils gegebenenfalls durch Nitro, Cyano, Halogen, C₁-C₄-Alkyl oder C₁-C₄-Alkoxy substituiertes Heterocyclyl oder Heterocyclylalkyl mit jeweils bis zu 6 Kohlenstoffatomen und zusätzlich 1 bis 4 Stickstoffatomen und/oder 1 bis 2 Sauerstoff- oder Schwefelatomen in der Heterocyclylgruppe und gegebenenfalls 1 bis 4 Kohlenstoffatomen im Alkylteil steht,
R² für Wasserstoff, Cyano, Nitro, Halogen, für jeweils gegebenenfalls durch Cyano, Halogen oder C₁-C₄-Alkoxy substituiertes Alkyl, Alkoxy, Alkoxycarbonyl, Alkylthio, Alkylsulfinyl oder Alkylsulfonyl mit jeweils 1 bis 6 Kohlenstoffatomen in der Alkylgruppe, oder für jeweils gegebenenfalls durch Cyano oder Halogen substituiertes Alkenyl, Alkinyl, Alkenyloxy oder Alkinyloxy mit jeweils 2 bis 6 Kohlenstoffatomen in der Alkenyl- oder Alkinylgruppe steht,
R³ für durch Halogen substituiertes Alkoxy mit 1 bis 6 Kohlenstoffatomen steht, und
R⁴ für Wasserstoff, Hydroxy, Amino, Cyano, für C₂-C₁₀-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes Alkyl mit 1 bis 6 Kohlenstoffatomen, für jeweils gegebenenfalls durch Fluor, Chlor und/oder Brom substituiertes Alkenyl oder Alkinyl mit jeweils 2 bis 6 Kohlenstoffatomen, für jeweils gegebenenfalls durch Fluor, Chlor, Brom, Cyano, C₁-C₄-Alkoxy oder C₁-C₄-Alkoxy-carbonyl substituiertes Alkoxy, Alkylamino oder Alkylcarbonylamino mit jeweils 1 bis 6 Kohlenstoffatomen in der Alkylgruppe, für Alkenyloxy mit 3 bis 6 Kohlenstoffatomen, für Dialkylamino mit jeweils 1 bis 4 Kohlenstoffatomen in den Alkylgruppen, für jeweils gegebenenfalls durch Fluor, Chlor, Brom, Cyano und/oder C₁-C₄-Alkyl substituiertes Cycloalkyl, Cycloalkylamino oder Cycloalkylalkyl mit jeweils 3 bis 6 Kohlenstoffatomen in der Alkylgruppe und gegebenenfalls 1 bis 4 Kohlenstoffatomen im Alkylteil, oder für jeweils gegebenenfalls durch Fluor, Chlor, Brom, Cyano, Nitro, C₁-C₄-Alkyl, Trifluormethyl und/oder C₁-C₄-Alkoxy substituiertes Aryl oder Arylalkyl mit jeweils 6 oder 10 Kohlenstoffatomen in der Arylgruppe und gegebenenfalls 1 bis 4 Kohlenstoffatomen im Alkylteil steht,
- sowie Salze der Verbindungen der Formel (I) -.

2. Verbindungen nach Anspruch 1, **dadurch gekennzeichnet, dass**
R¹ für jeweils gegebenenfalls durch Cyano, Fluor, Chlor, Methoxy oder Ethoxy substituiertes Methyl, Ethyl, n- oder i-Propyl, n-, i-, s- oder t-Butyl, für jeweils gegebenenfalls durch Cyano, Fluor oder Chlor substituiertes Propenyl, Butenyl, Propinyl oder Butinyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls durch Cyano, Fluor, Chlor, Methyl, Ethyl, n- oder i-Propyl, Trifluormethyl, Methoxy, Ethoxy, n- oder i-Propoxy, Difluormethoxy oder Trifluormethoxy substituiertes Phenyl, Phenylmethyl oder Phenylethyl, oder für jeweils gegebenenfalls durch Cyano, Fluor, Chlor, Brom, Methyl, Ethyl, n- oder i-Propyl, Methoxy, Ethoxy, n- oder i-Propoxy substituiertes Heterocyclyl oder Heterocyclylmethyl steht, wobei die Heterocyclylgruppe jeweils aus der Reihe Oxetanyl, Thietanyl, Furyl, Tetrahydrofuryl, Thienyl, Tetrahydrothienyl ausgewählt ist,
R² für Wasserstoff, Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder 1-Propoxy, Methoxycarbonyl, Ethoxycarbonyl, n- oder i-Propoxycarbonyl, Methylthio, Ethylthio, n- oder i-Propylthio, Methylsulfinyl, Ethylsulfinyl, Methylsulfonyl oder Ethylsulfonyl, oder für jeweils gegebenenfalls durch Cyano, Fluor oder Chlor substituiertes Propenyl, Butenyl, Propinyl, Butinyl, Propenyloxy, Butenyloxy, Propinyloxy oder Butinyloxy steht,
R³ für jeweils durch Fluor oder Chlor substituiertes Methoxy, Ethoxy, n-oder i-Propoxy, n-, i-, s- oder t-Butoxy, n-, i-, s- oder t-Pentyloxy, oder neo-Pentyloxy steht, und
R⁴ für Wasserstoff, Hydroxy, Amino, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor und/oder Brom substituiertes Ethenyl, Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylamino, Ethylamino, n- oder i-Propylamino, n-, i-, s- oder t-Butylamino, für Propenyloxy oder Butenyloxy, für Dimethylamino oder Diethylamino, für jeweils gegebenenfalls durch Fluor, Chlor, Methyl und/oder Ethyl substituiertes Cyclopropyl, Cyclobutyl, Cyclopentyl, Cyclohexyl, Cyclopropylamino, Cyclobutylamino, Cyclopentylamino, Cyclohexylamino, Cyclopropylmethyl, Cyclobutylmethyl, Cyclopentylmethyl oder Cyclohexylmethyl, oder für jeweils gegebenenfalls durch Fluor, Chlor, Methyl, Trifluormethyl und/oder Methoxy substituiertes Phenyl oder Benzyl steht.

3. Verbindungen gemäß Anspruch 1, **dadurch gekennzeichnet, dass**
R¹ für jeweils gegebenenfalls durch Fluor, Chlor, Methoxy oder Ethoxy substituiertes Methyl, Ethyl, n- oder i-Propyl steht,
R² für Fluor, Chlor, Brom oder für jeweils gegebenenfalls durch Fluor, Chlor, Methoxy oder Ethoxy substituiertes Methyl, Ethyl, n- oder i-Propyl steht,
R³ für jeweils durch Fluor oder Chlor substituiertes Methoxy, Ethoxy, n-oder i-Propoxy, n-, i-, s- oder t-Butoxy, n-, i-, s- oder t-Pentyloxy oder neo-Pentyloxy, steht, und
R⁴ für jeweils gegebenenfalls durch Fluor, Chlor, Methoxy oder Ethoxy substituiertes Methyl, Ethyl, n- oder i-Propyl, für jeweils gegebenenfalls durch Fluor oder Chlor substituiertes Ethenyl, Propenyl oder Propinyl, für jeweils gegebenenfalls durch Fluor, Chlor, Methoxy oder Ethoxy substituiertes Methoxy, Ethoxy, n- oder i-Propoxy, für Methylamino, oder für Cyclopropyl steht.

4. Verbindungen gemäß Anspruch 3, **dadurch gekennzeichnet, dass** R¹ für Methyl steht.

5. Verbindungen gemäß Anspruch 3, **dadurch gekennzeichnet, dass** R¹ für Ethyl steht.

6. Verbindungen gemäß Anspruch 3, **dadurch gekennzeichnet, dass** R¹ für n-Propyl steht.

7. Verbindungen gemäß Anspruch 3, **dadurch gekennzeichnet, dass** R¹ für i-Propyl steht.

8. Verfahren zum Herstellen der Verbindungen gemäß Anspruch 1, **dadurch gekennzeichnet, dass**
(a) substituierte Thiophen-3-sulfonamide der allgemeinen Formel (II) 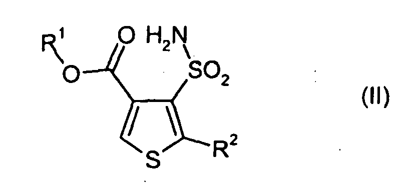 in welcher
R¹ und R² die in Anspruch 1 angegebene Bedeutung haben,
mit substituierten Triazolin(thi)onen der allgemeinen Formel (III) 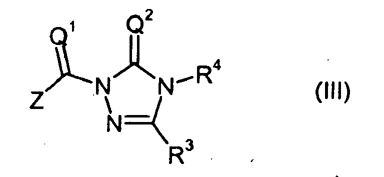 in welcher
Q¹, Q², R³ und R⁴ die in Anspruch 1 angegebene Bedeutung haben und
Z für Halogen, Alkoxy, Aryloxy oder Arylalkoxy steht,
gegebenenfalls in Gegenwart eines Reaktionshilfsmittels und gegebenenfalls in Gegenwart eines Verdünnungsmittels umgesetzt werden,
oder dass
(b) substituierte Thien-3-yl-sulfonyl-iso(thio)cyanate der allgemeinen Formel (IV) 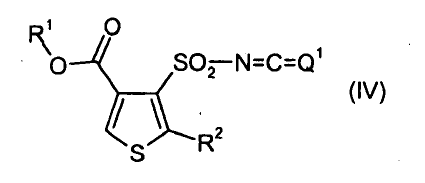 in welcher
Q¹, R¹ und R² die IN Anspruch 1 angegebene Bedeutung haben,
mit Triazolin(thi)onen der allgemeinen Formel (V) 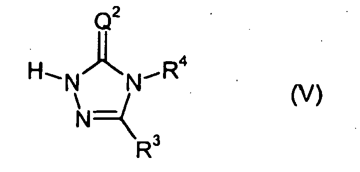 in welcher
Q², R⁴ und R⁵ die in Anspruch 1 angegebene Bedeutung haben, gegebenenfalls in Gegenwart eines Reaktionshilfsmittels und gegebenenfalls in Gegenwart eines Verdünnungsmittels umgesetzt werden,
oder dass
(c) substituierte Thiophen-3-sulfonsäurechloride der allgemeinen Formel (VI) 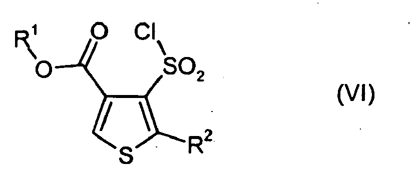 in welcher
R¹ und R² die in Anspruch 1 angegebene Bedeutung haben,
mit Triazolin(thi)onen der allgemeinen Formel (V) 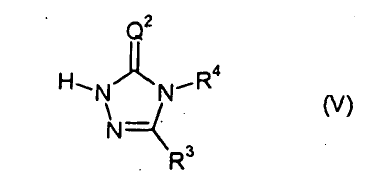 in welcher
Q² , R⁴ und R⁵ die in Anspruch 1 angegebene Bedeutung haben,
und Metall(thio)cyanaten der allgemeinen Formel (VII)
M-Q¹-CN (VII)
in welcher
Q¹ die oben angegebene Bedeutung hat,
gegebenenfalls in Gegenwart eines Reaktionshilfsmittels und gegebenenfalls in Gegenwart eines Verdünnungsmittels umgesetzt werden,
oder dass
(d) substituierte Thiophen-3-sulfonsäurechloride der allgemeinen Formel (VI) 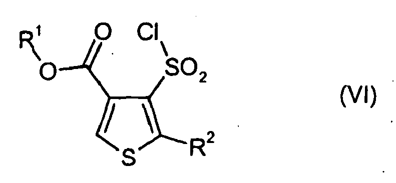 in welcher
R¹ und R² die in Anspruch 1 angegebene Bedeutung haben,
mit Triazolin(thi)on-(thio)carboxamiden der allgemeinen Formel (VIII) 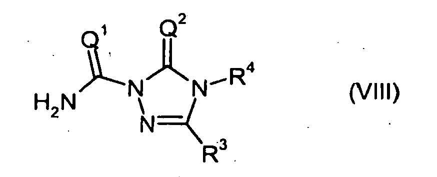 in welcher
Q¹, Q², R³ und R⁴ die in Anspruch 1 angegebene Bedeutung haben,
gegebenenfalls in Gegenwart eines Reaktionshilfsmittels und gegebenenfalls in Gegenwart eines Verdünnungsmittels umgesetzt werden,
oder dass
(e) substituierte Thien-3-yl-sulfonylamino(thio)carbonylverbindungen der allgemeinen Formel (IX) 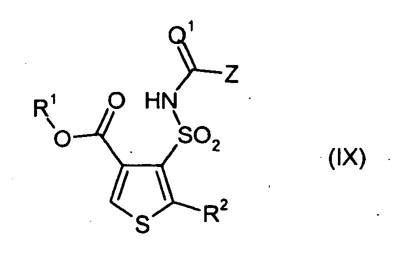
in welcher
Q¹, R¹ und R² die in Anspruch 1 angegebene Bedeutung haben und
Z für Halogen, Alkoxy, Aryloxy oder Arylalkoxy steht,
mit Triazolin(thi)onen der allgemeinen Formel (V) 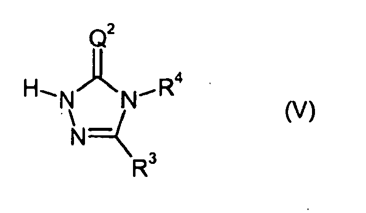 in welcher
Q², R⁴ und R⁵ die in Anspruch 1 angegebene Bedeutung haben, gegebenenfalls in Gegenwart eines Reaktionshilfsmittels und gegebenenfalls in Gegenwart eines Verdünnungsmittels umgesetzt werden,
und gegebenenfalls die nach den Verfahren (a), (b), (c), (d) oder (e) erhaltenen Verbindungen der Formel (I) nach üblichem Methoden in Salze überführt werden.

9. Verwendung von mindestens einer Verbindung gemäß einem der Ansprüche 1 bis 8 zum Bekämpfen von unerwünschten Pflanzen.

10. Herbizides Mittel, **gekennzeichnet durch** einen Gehalt an einer Verbindung gemäß einem der Ansprüche 1 bis 8 und üblichen Streckmitteln und/oder oberflächenaktiven Mitteln.

11. Verfahren zum Bekämpfen von unerwünschtem Pflanzenwuchs, **dadurch gekennzeichnet, dass** man mindestens eine Verbindung gemäß einem der Ansprüche 1 bis 8 auf die unerwünschten Pflanzen und/oder ihren Lebensraum einwirken lässt.

## Claims

1. Compounds of the general formula (I) 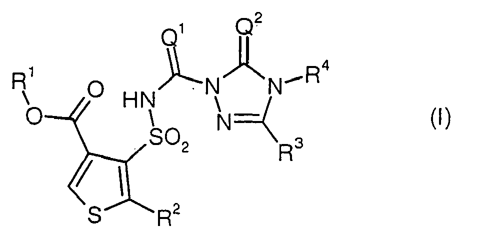 in which
Q¹ represents 0 (oxygen) or S (sulphur),
Q² represents O (oxygen) or S (sulphur),
R¹ represents optionally cyano-, halogen- or C₁-C₄-alkoxy-substituted alkyl having 1 to 6 carbon atoms, represents in each case optionally cyano- or halogen-substituted alkenyl or alkinyl having in each case 2 to 6 carbon atoms, represents in each case optionally cyano-, halogen- or C₁-C₄-alkyl- substituted cycloalkyl or cycloalkylalkyl having in each case 3 to 6 carbon atoms in the cycloalkyl group and optionally 1 to 4 carbon atoms in the alkyl moiety, represents in each case optionally nitro-, cyano-, halogen-, C₁-C₄-alkyl- or C₁-C₄-alkoxy-substituted aryl or arylalkyl having in each case 6 or 10 carbon atoms in the aryl group and optionally 1 to 4 carbon atoms in the alkyl moiety, or represents in each case optionally nitro-, cyano-, halogen-, C₁-C₄-alkyl- or C₁-C₄-alkoxy-substituted heterocyclyl or heterocyclylalkyl having in each case up to 6 carbon atoms and additionally 1 to 4 nitrogen atoms and/or 1 to 2 oxygen or sulphur atoms in the heterocyclyl group and optionally 1 to 4 carbon atoms in the alkyl moiety,
R² represents hydrogen, cyano, nitro, halogen, represents in each case optionally cyano-, halogen- or C₁-C₄-alkoxy-substituted alkyl, alkoxy, alkoxycarbonyl, alkylthio, alkylsulphinyl or alkylsulphonyl having in each case 1 to 6 carbon atoms in the alkyl group, or represents in each case optionally cyano- or halogen-substituted alkenyl, alkinyl, alkenyloxy or alkinyloxy having in each case 2 to 6 carbon atoms in the alkenyl or alkinyl group,
R³ represents halogen-substituted alkoxy having 1 to 6 carbon atoms, and
R⁴ represents hydrogen, hydroxyl, amino, cyano, represents C₂-C₁₀-alkylideneamino, represents optionally fluorine-, chlorine-, bromine-, cyano-, C₁-C₄-alkoxy-, C₁-C₄-alkyl-carbonyl- or C₁-C₄-alkoxy-carbonyl-substituted alkyl having 1 to 6 carbon atoms, represents in each case optionally fluorine-, chlorine- and/or bromine-substituted alkenyl or alkinyl having in each case 2 to 6 carbon atoms, represents in each case optionally fluorine-, chlorine-, bromine-, cyano-, C₁-C₄-alkoxy- or C₁-C₄-alkoxy-carbonyl-substituted alkoxy, alkylamino or alkyl-carbonylamino having in each case 1 to 6 carbon atoms in the alkyl group, represents alkenyloxy having 3 to 6 carbon atoms, represents dialkylamino having in each case 1 to 4 carbon atoms in the alkyl groups, represents in each case optionally fluorine-, chlorine-, bromine-, cyano- and/or C₁-C₄-alkyl-substituted cycloalkyl, cycloalkylamino or cycloalkylalkyl having in each case 3 to 6 carbon atoms in the alkyl group and optionally 1 to 4 carbon atoms in the alkyl moiety, or represents in each case optionally fluorine-, chlorine-, bromine-, cyano-, nitro-, C₁-C₄-alkyl-, trifluoromethyl-and/or C₁-C₄-alkoxy-substituted aryl or arylalkyl having in each case 6 or 10 carbon atoms in the aryl group and optionally 1 to 4 carbon atoms in the alkyl moiety,
- and the salts of the compounds of the formula (I) .

2. Compounds according to Claim 1, **characterized in that**
R¹ represents in each case optionally cyano-, fluorine-, chlorine-, methoxy- or ethoxy-substituted methyl, ethyl, n- or i-propyl, n-, i-, s- or t-butyl, represents in each case optionally cyano-, fluorine- or chlorine-substituted propenyl, butenyl, propinyl or butinyl, represents in each case optionally cyano-, fluorine-, chlorine-, methyl- or ethyl-substituted cyclopropyl, cyclobutyl, cyclopentyl, cyclohexyl, cyclopropylmethyl, cyclobutylmethyl, cyclopentylmethyl or cyclohexylmethyl, represents in each case optionally cyano-, fluorine-, chlorine-, methyl-, ethyl-, n- or i-propyl-, trifluoromethyl-, methoxy-, ethoxy-, n- or i-propoxy-, difluoromethoxy- or trifluoromethoxy-substituted phenyl, phenylmethyl or phenylethyl, or represents in each case optionally cyano-, fluorine-, chlorine-, bromine-, methyl-, ethyl-, n- or i-propyl-, methoxy-, ethoxy-, n- or i-propoxy-substituted heterocyclyl or heterocyclylmethyl, where the heterocyclyl group is in each case selected from the group consisting of oxetanyl, thietanyl, furyl, tetrahydrofuryl, thienyl, tetrahydrothienyl,
R² represents hydrogen, cyano, fluorine, chlorine, bromine, represents in each case optionally cyano-, fluorine-, chlorine-, methoxy- or ethoxy-substituted methyl, ethyl, n- or i-propyl, n-, i-, s- or t-butyl, methoxy, ethoxy, n- or i-propoxy, methoxycarbonyl, ethoxycarbonyl, n- or i-propoxycarbonyl, methylthio, ethylthio, n- or i-propylthio, methylsulphinyl, ethylsulphinyl, methylsulphonyl or ethylsulphonyl, or represents in each case optionally cyano-, fluorine- or chlorine-substituted propenyl, butenyl, propinyl, butinyl, propenyloxy, butenyloxy, propinyloxy or butinyloxy,
R³ represents in each case fluorine- or chlorine-substituted methoxy, ethoxy, n- or i-propoxy, n-, i-, s- or t-butoxy, n-, i-, s- or t-pentyloxy, or neopentyloxy, and
R⁴ represents hydrogen, hydroxyl, amino, represents in each case optionally fluorine-, chlorine-, cyano-, methoxy- or ethoxy-substituted methyl, ethyl, n- or i-propyl, n-, i-, s- or t-butyl, represents in each case optionally fluorine-, chlorine- and/or bromine-substituted ethenyl, propenyl, butenyl, propinyl or butinyl, represents in each case optionally fluorine-, chlorine-, cyano-, methoxy- or ethoxy-substituted methoxy, ethoxy, n- or i-propoxy, n-, i-, s- or t-butoxy, methylamino, ethylamino, n- or i-propylamino, n-, i-, s- or t-butylamino, represents propenyloxy or butenyloxy, represents dimethylamino or diethylamino, represents in each case optionally fluorine-, chlorine-, methyl- and/or ethyl-substituted cyclopropyl, cyclobutyl, cyclopentyl, cyclohexyl, cyclopropylamino, cyclobutylamino, cyclopentylamino, cyclohexylamino, cyclopropylmethyl, cyclobutylmethyl, cyclopentylmethyl or cyclohexylmethyl, or represents in each case optionally fluorine-, chlorine-, methyl-, trifluoromethyl- and/or methoxy-substituted phenyl or benzyl.

3. Compounds according to Claim 1, **characterized in that**
R¹ represents in each case optionally fluorine-, chlorine-, methoxy- or ethoxy-substituted methyl, ethyl, n- or i-propyl,
R² represents fluorine, chlorine, bromine or represents in each case optionally fluorine-, chlorine-, methoxy- or ethoxy-substituted methyl, ethyl, n- or i-propyl,
R³ represents in each case fluorine- or chlorine-substituted methoxy, ethoxy, n- or i-propoxy, n-, i-, s- or t-butoxy, n-, i-, s- or t-pentyloxy or neopentyloxy, and
R⁴ represents in each case optionally fluorine-, chlorine-, methoxy- or ethoxy-substituted methyl, ethyl, n- or i-propyl, represents in each case optionally fluorine- or chlorine-substituted ethenyl, propenyl or propinyl, represents in each case optionally fluorine-, chlorine-, methoxy- or ethoxy-substituted methoxy, ethoxy, n- or i-propoxy, represents methylamino, or represents cyclopropyl.

4. Compounds according to Claim 3, **characterized in that** R¹ represents methyl.

5. Compounds according to Claim 3, **characterized in that** R¹ represents ethyl.

6. Compounds according to Claim 3, **characterized in that** R¹ represents n-propyl.

7. Compounds according to Claim 3, **characterized in that** R¹ represents i-propyl.

8. Process for the preparation of compounds according to Claim 1, **characterized in that**
(a) substituted thiophene-3-sulphonamides of the general formula (II) 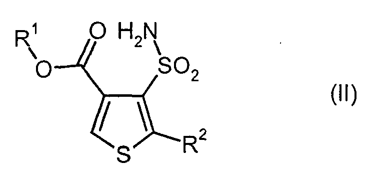 in which
R¹ and R² are each as defined in Claim 1,
are reacted with substituted triazolin(ethi)ones of the general formula (III) 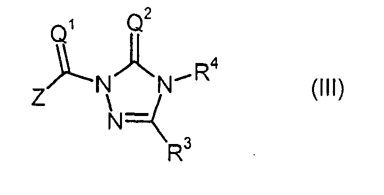 in which
Q¹, Q², R³ and R⁴ are each as defined in Claim 1 and
Z represents halogen, alkoxy, aryloxy or arylalkoxy,
if appropriate in the presence of a reaction auxiliary and if appropriate in the presence of a diluent,
or that
(b) substituted thien-3-yl-sulphonyl iso(thio)-cyanates of the general formula (IV) 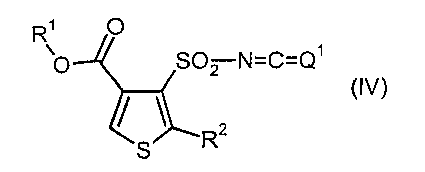 in which
Q¹, R¹ and R² are each as defined in Claim 1,
are reacted with triazolin(ethi)ones of the general formula (V) 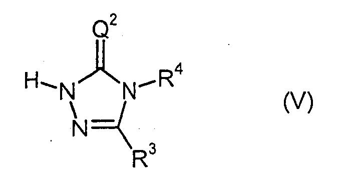 in which
Q², R⁴ and R⁵ are each as defined in Claim 1, if appropriate in the presence of a reaction auxiliary and if appropriate in the presence of a diluent,
or that
(c) substituted thiophene-3-sulphonyl chlorides of the general formula (VI) 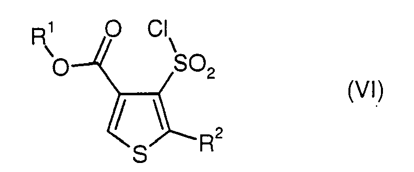 in which
R¹ and R² are each as defined in Claim 1,
are reacted with triazolin(ethi)ones of the general formula (V) 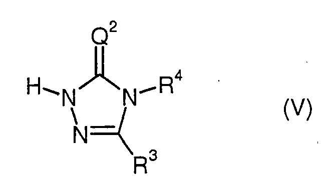 in which
Q², R⁴ and R⁵ are each as defined in Claim 1,
and metal (thio)cyanates of the general formula (VII)
M-Q¹-CN (VII)
in which
Q¹ is as defined above,
if appropriate in the presence of a reaction auxiliary and if appropriate in the presence of a diluent,
or that
(d) substituted thiophene-3-sulphonyl chlorides of the general formula (VI) 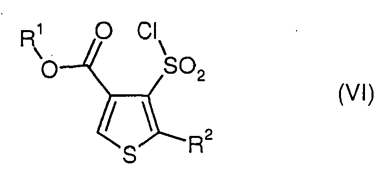 in which
R¹ and R² are each as defined in Claim 1, are reacted with triazolin(ethi)one-(thio)-carboxamides of the general formula (VIII) 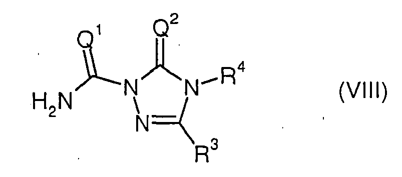 in which
Q¹, Q², R³ and R⁴ are each as defined in Claim 1,
if appropriate in the presence of a reaction auxiliary and if appropriate in the presence of a diluent,
or that
(e) substituted thien-3-yl-sulphonylamino(thio)-carbonyl compounds of the general formula (IX) 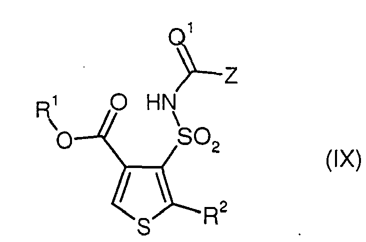
in which
Q¹, R¹ and R² are each as defined in Claim 1 and
Z represents halogen, alkoxy, aryloxy or arylalkoxy,
are reacted with triazolin(ethi)ones of the general formula (V) 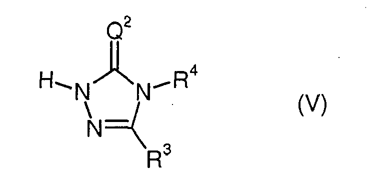 in which
Q², R⁴ and R⁵ are each as defined in Claim 1,
if appropriate in the presence of a reaction auxiliary and if appropriate in the presence of a diluent,
and the compounds of the formula (I) obtained by the processes (a), (b), (c), (d) or (e) are, if appropriate, converted by customary methods into salts.

9. Use of at least one compound according to any of Claims 1 to 8 for controlling undesirable plants.

10. Herbicidal compositions, **characterized in that** they comprise a compound according to any of Claims 1 to 8 and customary extenders and/or surfactants.

11. Method for controlling undesirable vegetation, **characterized in that** at least one compound according to any of Claims 1 to 8 is allowed to act on undesirable plants and/or their habitat.

## Revendications

1. Composés de formule générale (I) 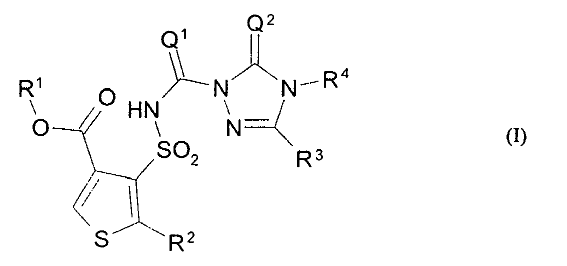 dans laquelle
Q¹ est O (oxygène) ou S (soufre),
Q² est O (oxygène) ou S (soufre),
R¹ est
- un radical alkyle, comprenant 1 à 6 atomes de carbone, éventuellement substitué par un radical cyano, un atome d'halogène ou alcoxy en C₁ à C₄; un radical alcényle ou alcynyle, comprenant respectivement 2 à 6 atomes de carbone, éventuellement substitué par un radical cyano ou halogène;
- un radical cycloalkyle ou cycloalkylalkyle, comprenant respectivement 3 à 6 atomes de carbone dans le radical cycloalkyle, éventuellement 1 à 4 atomes de carbone dans la partie alkyle, éventuellement substitué par un radical cyano, un atome d'halogène ou alkyle en C₁ à C₄;
- un radical aryle ou arylalkyle, comprenant respectivement 6 ou 10 atomes de carbone dans le radical aryle et éventuellement 1 à 4 atomes de carbone dans la partie alkyle, éventuellement substitué par un radical nitro, cyano, un atome d'halogène, alkyle en C₁ à C₄ ou alcoxy en C₁ à C₄;
- ou un radical hétérocyclyle ou hétérocyclylalkyle, comprenant respectivement jusqu'à 6 atomes de carbone et en plus 1 à 4 atomes d'azote et/ou 1 à 2 atomes d'oxygène ou de soufre dans le radical hétérocyclyle et éventuellement 1 à 4 atomes de carbone dans la partie alkyle, éventuellement substitué par un radical nitro, cyano, un atome d'halogène, alkyle en C₁ à C₄ ou alcoxy en C₁ à C₄,
R² est
un atome d'hydrogène; un groupe cyano; un groupe nitro; un atome d'halogène; un radical alkyle, alcoxy, alcoxycarbonyle, alkylthio, alkylsulfinyle ou alkylsulfonyle, comprenant respectivement 1 a 6 atomes de carbone dans le radical alkyle, éventuellement substitué par un radical cyano, un atome d'halogène ou un radical alcoxy en C₁ à C₄; ou
- un radical alcényle, alcynyle, alcényloxy ou alcynyloxy comprenant respectivement 2 à 6 atomes de carbone dans le radical alcényle ou alcynyle, éventuellement substitué par un radical cyano ou halogène,
R³ est un radical alcoxy de 1 à 6 atomes de carbone substitué par un halogène, et
R⁴ est
- un atome d'hydrogène;
- un groupe hydroxy; amino; cyano; alkylidènenamino en C₂ à C₁₀;
- un radical alkyle comprenant 1 à 6 atomes de carbone éventuellement substitué par un groupe fluoro, chloro, bromo, cyano, alcoxy en C₁ à C₄, alkylcarbonyle en C₁ à C₄ ou alcoxycarbonyle en C₁ à C₄;
- un radical alcényle ou alcynyle, comprenant respectivement 2 à 6 atomes de carbone, éventuellement substitué par un groupe fluoro, chloro et/ou bromo;
- un radical alcoxy, alkylamino ou alkylcarbonylamino comprenant respectivement 1 à 6 atomes de carbone dans le radical alkyle, éventuellement substitué par un groupe fluoro, chloro, bromo, cyano, alcoxy en C₁ à C₄ ou alcoxycarbonyle en C₁ à C₄;
- un radical alcényloxy de 3 à 6 atomes de carbone; un radical dialkylamino comprenant 1 à 4 atomes de carbone dans chacun des radicaux alkyle;
- un radical cycloalkyle, cycloalkylamino ou cycloalkylalkyle, comprenant respectivement 3 à 6 atomes de carbone dans le radical alkyle et éventuellement 1 à 4 atomes de carbone dans la partie alkyle, éventuellement substitué par un groupe fluoro, chloro, bromo, cyano et/ou alkyle en C₁ à C₄;
- ou un radical aryle ou arylalkyle, comprenant respectivement 6 ou 10 atomes de carbone dans le radical aryle et éventuellement 1 à 4 atomes de carbone dans la partie alkyle, éventuellement substitué par un groupe fluoro, chloro, bromo, cyano, nitro, alkyle en C₁ à C₄, trifluorométhyle et/ou alcoxy en C₁ à C₄,
ainsi que des sels des composés de formule (I).

2. Composés selon la revendication 1, **caractérisés en ce que**
R¹ est
- un radical méthyle, éthyle, *n*- ou *i*-propyle, *n*-, *i-, s*- ou *t*-butyle respectivement substitué éventuellement par un radical cyano, fluoro, chloro, méthoxy ou éthoxy;
- un radical propényle, butényle, propynyle ou butynyle respectivement substitué éventuellement par un radical cyano, fluoro ou chloro;
- un radical cyclopropyle, cyclobutyle, cyclopentyle, cyclohexyle, cyclopropylméthyle, cyclobutylméthyle, cyclopentylméthyle ou cyclohexylméthyle respectivement substitué éventuellement par un radical cyano, fluoro, chloro, méthyle ou éthyle;
- un radical phényle, phénylméthyle ou phényléthyle respectivement substitué éventuellement par un radical cyano, fluoro, chloro, méthyle, éthyle, *n*- ou *i*-propyle, trifluorométhyle, méthoxy, éthoxy, n-ou i-propoxy, difluorométhoxy ou trifluorométhoxy;
- ou un radical hétérocyclyle ou hétérocyclylméthyle respectivement substitué éventuellement par un radical cyano, fluoro, chloro, bromo, méthyle, éthyle, *n*- ou *i*-propyle, méthoxy, éthoxy, *n*- ou *i*-propoxy, le radical hétérocyclyle étant choisi à chaque fois dans la série, comprenant les oxétanyle, thiétanyle, furyle, tétrahydrofuryle, thiényle, tétrahydrothiényle,
R² est
- un atome d'hydrogène ;
- un groupe cyano;
- un atome de fluor; de chlore; de brome;
- un radical méthyle, éthyle, *n*- ou *i*-propyle, *n*-, *i-, s*- ou *t*-butyle, méthoxy, éthoxy, *n*- ou *i*-propoxy, méthoxycarbonyle, éthoxycarbonyle, *n*- ou *i*-propoxycarbonyle, méthylthio, éthylthio, n-ou *i*-propylthio, méthylsulfinyle, éthylsulfinyle, méthylsulfonyle ou éthylsulfonyle respectivement substitué éventuellement par un radical cyano, fluoro, chloro, méthoxy ou éthoxy; ou un radical propényle, butényle, propynyle, butynyle, propényloxy, butényloxy, propynyloxy ou butynyloxy respectivement substitué éventuellement par un radical cyano, fluoro ou chloro,
R³ est un radical méthoxy, éthoxy, *n*- ou *i*-propoxy, *n*-, *i-, s*- ou *t*-butoxy, *n-, i-, s*- ou *t*-pentyloxy ou néo-pentyloxy respectivement substitué par un groupe fluoro ou chloro, et
R⁴ est
- un atome d'hydrogène;
- un groupe hydroxy; amino;
- un radical méthyle, éthyle, *n*- ou *i*-propyle, *n-, i-, s*- ou *t*-butyle respectivement substitué éventuellement par un groupe fluoro, chloro, cyano, méthoxy ou éthoxy;
- un radical éthényle, propényle, butényle, propynyle ou butynyle respectivement substitué éventuellement par un groupe fluoro, chloro et/ou bromo;
- un radical méthoxy, éthoxy, *n*- ou *i*-propoxy, *n-, i-, s*- ou *t*-butoxy, méthylamino, éthylamino, *n*- ou *i*-propylamino, *n-, i-, s*- ou *t*-butylamino respectivement substitué éventuellement par un groupe fluoro, chloro, cyano, méthoxy ou éthoxy;
- un radical propényloxy ou butényloxy;
- un radical diméthylamino ou diéthylamino;
- un radical cyclopropyle, cyclobutyle, cyclopentyle, cyclohexyle, cyclopropylamino, cyclobutylamino, cyclopentylamino, cyclohexylamino, cyclopropylméthyle, cyclobutylméthyle, cyclopentylméthyle ou cyclohexylméthyle respectivement substitué éventuellement par un groupe fluoro, chloro, méthyle et/ou éthyle;
- ou un radical phényle ou benzyle, respectivement substitué éventuellement par un groupe fluoro, chloro, méthyle, trifluorométhyle et/ou méthoxy.

3. Composés selon la revendication 1, **caractérisés en ce que**
R¹ est un radical méthyle, éthyle, *n*- ou *i*-propyle respectivement substitué éventuellement par un groupe fluoro, chloro, méthoxy ou éthoxy,
R² est un atome de fluor, de chlore, de brome ou un radical méthyle, éthyle, *n*- ou *i*-propyle respectivement substitué éventuellement par un groupe fluoro, chloro, méthoxy ou éthoxy,
R³ est un radical méthoxy, éthoxy, *n*- ou *i*-propoxy, *n*-, *i-, s*- ou *t*-butoxy, *n*-, *i-, s*- ou *t*-pentyloxy ou néo-pentyloxy, respectivement substitué par un groupe fluoro ou chloro, et
R⁴ est
- un radical méthyle, éthyle, *n*- ou *i*-propyle respectivement substitué éventuellement par un groupe fluoro, chloro, méthoxy ou éthoxy;
- un radical éthényle, propényle ou propynyle respectivement substitué éventuellement par un groupe fluoro ou chloro;
- un radical méthoxy, éthoxy, *n*- ou *i*-propoxy respectivement substitué éventuellement par un groupe fluoro, chloro, méthoxy ou éthoxy;
- un radical méthylamino; ou un radical cyclopropyle.

4. Composés selon la revendication 3, **caractérisés en ce que** R¹ est le radical méthyle.

5. Composés selon la revendication 3, **caractérisés en ce que** R¹ est le radical éthyle.

6. Composés selon la revendication 3, **caractérisés en ce que** R¹ est le raical *n-*propyle.

7. Composés selon la revendication 3, **caractérisés en ce que** R¹ est le radical *i-*propyle.

8. Procédé de préparation des composés selon la revendication 1, **caractérisé en ce que** l'on fait réagir
(a)des thiophène-3-sulfonamides substitués de formule générale (II) 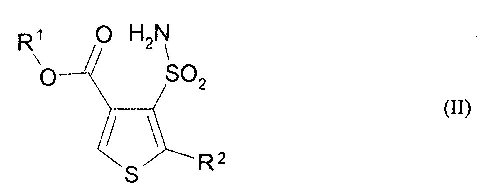 dans laquelle
R¹ et R² ont la signification indiquée dans la revendication 1, avec des triazolin(thi)ones substituées de formule générale (III) 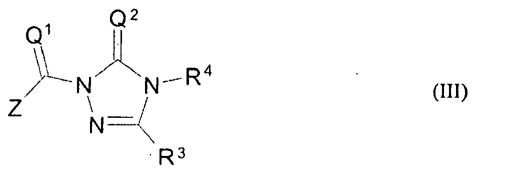 dans laquelle
Q¹, Q², R³ et R⁴ ont la signification indiquée dans la revendication 1 et
Z est un atome d'halogène, un radical alcoxy, aryloxy ou arylalcoxy, éventuellement en présence d'un auxiliaire réactionnel et éventuellement en présence d'un diluant,
ou **en ce que** l'on fait réagir
(b)des iso(thio)cyanates de thién-3-ylsulfonyle substitués de formule générale (IV) 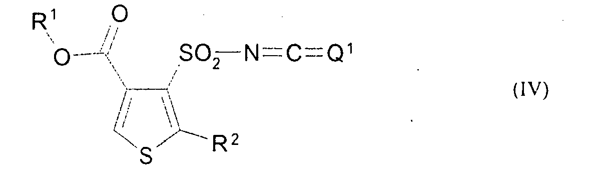 dans laquelle
Q¹, R¹ et R² ont la signification indiquée dans la revendication 1,
avec des triazolin(thi)ones de formule générale (V) 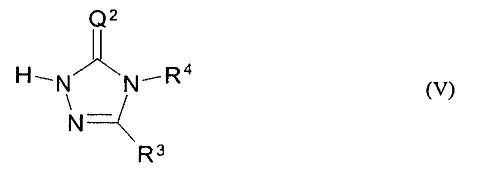 dans laquelle
Q², R⁴ et R⁵ ont la signification indiquée dans la revendication 1, éventuellement en présence d'un auxiliaire réactionnel et éventuellement en présence d'un diluant,
ou **en ce que** l'on fait réagir
(c)des chlorures d'acide thiophène-3-sulfonique substitués de formule générale (VI) 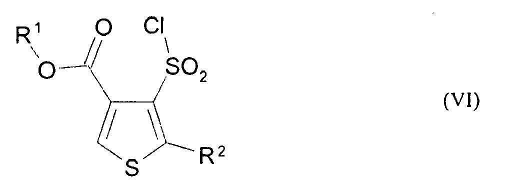 dans laquelle
R¹ et R² ont la signification indiquée dans la revendication 1, avec des triazolin(thi)ones de formule générale (V) 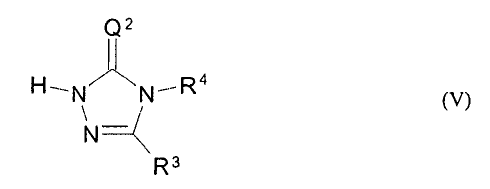 dans laquelle
Q², R⁴ et R⁵ ont la signification indiquée dans la revendication 1, et des (thio)cyanates métalliques de formule générale (VII)
M-Q¹-CN (VII)
dans laquelle
Q¹ a la signification indiquée plus haut, éventuellement en présence d'un auxiliaire réactionnel et éventuellement en présence d'un diluant,
ou **en ce que** l'on fait réagir
(d)des chlorures d'acide thiophène-3-sulfonique substitués de formule générale (VI) 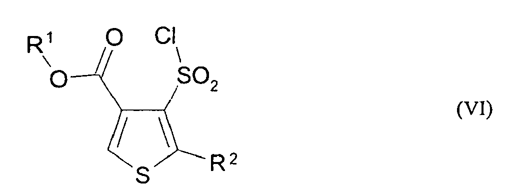 dans laquelle
R¹ et R² ont la signification indiquée dans la revendication 1, avec des (thio)carboxamides de triazolin(thi)ones de formule générale (VIII) 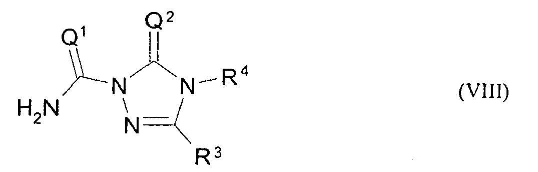 dans laquelle
Q¹, Q², R³ et R⁴ ont la signification indiquée dans la revendication 1, éventuellement en présence d'un auxiliaire réactionnel et éventuellement en présence d'un diluant,
ou **en ce que** l'on fait réagir
(e)des composés de thién-3-yl-sulfonylamino(thio)carbonyle substitués de formule générale (IX) 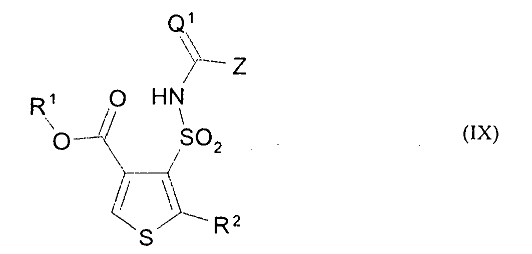 dans laquelle
Q¹, R¹ et R² ont la signification indiquée dans la revendication 1 et
Z est un atome d'halogène, un radical alcoxy, aryloxy ou arylalcoxy, avec des triazolin(thi)ones de formule générale (V) 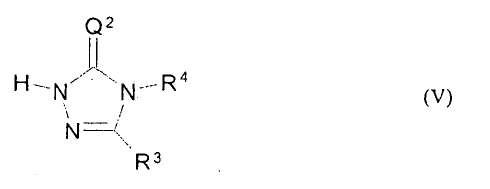 dans laquelle
Q², R⁴ et R⁵ ont la signification indiquée dans la revendication 1, éventuellement en présence d'un auxiliaire réactionnel et éventuellement en présence d'un diluant, et, éventuellement, on convertit en sels les composés de formule (I) obtenus par les procédés (a), (b), (c), (d) ou (e) par des méthodes usuelles.

9. Utilisation d'au moins un composé selon l'une des revendications 1 à 8 pour lutter contre des plantes indésirables.

10. Agent herbicide, **caractérisé en ce qu'**il contient un composé selon l'une des revendications 1 à 8 et des agents d'expansion et/ou des agents tensio-actifs usuels.

11. Procédé de lutte contre la croissance de plantes indésirables, **caractérisé en ce que** l'on fait agir au moins un composé selon l'une des revendications 1 à 8 sur les plantes indésirables et/ou sur leur biotope.
